# EUROPÄISCHE PATENTSCHRIFT

(11) **EP 2 214 724 B1**
(45) Veröffentlichungstag und Bekanntmachung des Hinweises auf die Patenterteilung: **25.12.2013**
(21) Anmeldenummer: 08874092.3
(22) Anmeldetag: 13.11.2008
(51) Int. Cl.: A61L 2/20, B65B 55/10, B67C 7/00, A61L 101/22

(54) **VORRICHTUNG UND VERFAHREN ZUM ERZEUGEN EINES STERILISIERENDEN GASGEMISCHS**
APPARATUS AND METHOD FOR THE PRODUCTION OF A STERILIZING GAS MIXTURE
DISPOSITIF ET PROCÉDÉ DE PRODUCTION D'UN MÉLANGE GAZEUX STÉRILISANT

(30) Priorität: 26.11.2007 DE 102007056833
(43) Veröffentlichungstag der Anmeldung: 11.08.2010
(73) Patentinhaber: GEA TDS GmbH, 31157 Sarstedt (DE)
(72) Erfinder: KOWALIK, Gottfried, 48712 Gescher (DE); ASSING, Hubert, 48683 Ahaus (DE); TACKE, Ludger, 46342 Velen (DE)
(86) Internationale Anmeldenummer: PCT/EP2008/009622
(87) Internationale Veröffentlichungsnummer: WO 2009/132686

(56) Entgegenhaltungen:
- WO-A-98/34649
- DE-A1- 3 540 161
- US-A- 2 242 466
- US-A- 4 512 935
- US-A- 4 537 749

## Beschreibung

### TECHNISCHES GEBIET

Die Erfindung bezieht sich auf eine Vorrichtung zum Erzeugen eines sterilisierenden Gasgemischs nach Anspruch 1, mit der das Gasgemisch aus einem gasförmigen ersten Bestandteil als kontinuierlicher Phase, insbesondere Luft wie Sterilluft, und einem flüssigen zweiten Bestandteil als disperser Phase, insbesondere einem Sterilisationsmittel wie Wasserstoffperoxid, in einem vorgegebenen Mengenverhältnis generiert wird und auf ein Verfahren zum Erzeugen eines sterilisierenden Gasgemischs nach Anspruch 19 sowie auf die Verwendung der Vorrichtung nach Anspruch 27. Die Erfindung ist insbesondere auf eine Vorrichtung und ein Verfahren zum Erzeugen eines Gemischs aus Luft und dampfförmigem Wasserstoffperoxid mit sterilisierenden Eigenschaften zur Verwendung in Formungs-, Abfüll- und Versiegelungs-Verpackungsmaschinen anwendbar.

### STAND DER TECHNIK

Werden Nahrungsmittel wie Milch, Saft, getrocknete und/oder zerkleinerte Nahrungsmittel, wie Suppe oder dergl. unter aseptischen Verpackungsbedingungen verpackt, dann können sie eine längere Zeit bei Raumtemperaturen gelagert werden, ohne zu verderben. Voraussetzung hierfür ist, dass das Nahrungsmittel keimfrei bereitgestellt und das Verpackungsmittel sowie das unmittelbare Umfeld, in dem das Abfüllen und Verpacken stattfinden, sterilisiert bzw. desinfiziert werden.

Die gewünschte Sterilisierung des Verpackungsmittels erfolgt beispielsweise in einer sog. Formungs-, Verpackungs- und Verschluss-Maschine wie der unter der Bezeichnung Typ 2450 Incoterms 2000 bekannten und von der Firma FEP, Ahaus, DE beziehbaren Verpackungsmaschine, in der beispielsweise das Verpackungsmittel ausgeformt und sterilisiert, das Produkt abgefüllt und das befüllte Verpackungsmittel mit einem gleichfalls sterilisierten Deckel versiegelt wird. Sämtliche Zonen der Verpackungsmaschine, die den vorgenannten Maßnahmen und Verfahrensschritten zugeordnet sind, werden in der Regel gleichfalls mit einem sterilisierenden Mittel beaufschlagt. Diesbezüglich wird ein Mittel bevorzugt, das als einphasiges Gasgemisch Luft und Wasserstoffperoxid (H₂O₂, im Folgenden stets H2O2 geschrieben) in Dampfform oder als zweiphasiges Aerosol die kontinuierliche Phase Luft und die disperse Phase flüssiges H2O2 in feinen Tröpfchen enthält. Es ist bekannt, das H2O2 mit Wasserdampf zu vermengen und das entstehende einphasige Gasgemisch dem Sterilisierungsbereich zuzuführen, wobei dort aufgrund der erhöhten Flüssigkeitsanteile die Gefahr der nicht gewollten Tröpfchenbildung besteht.

Die hohe bakterizide Wirkung von H2O2 beruht darauf, dass es gegenüber den meisten Stoffen ein sehr starkes Oxidationsmittel darstellt und damit als starkes Bleich- und Desinfektionsmittel fungiert. H2O2 ist eine blassblaue, in verdünnter Form farblose, weitgehend stabile Flüssigverbindung aus Wasserstoff und Sauerstoff. Bei der Verwendung in den vorg. Verpackungsmaschinen und zu Zwecken der Sterilisation und Desinfektion in der Lebensmittelindustrie allgemein wird es in einer Konzentration von 30 bis 35 % verwendet.

Aseptische Verpackungsbedingungen werden allerdings nur dann sichergestellt, wenn auf den zu sterilisierenden bzw. desinfizierenden Oberflächen ein geschlossener Flüssigkeitsfilm gebildet wird. Dies wird immer dann erreicht, wenn das H2O2 aus der Dampfphase an den kühleren Oberflächen der Verpackungsmittel und/oder den Behälterwänden der Verpackungszonen kondensiert. Hierzu ist zwingend H2O2 in Dampfform erforderlich, das zusammen mit einem Trägergas, wie beispielsweise Luft oder Sterilluft, ausgebracht wird. Das desinfizierende Gasgemisch, bestehend aus dem gasförmigen ersten Bestandteil, der Luft bzw. Sterilluft, und dem dampfförmigen zweiten Bestandteil, dem H2O2 in Dampfform, muss demzufolge bei einer Temperatur bereitgestellt werden, die unter den gegebenen Zustandsbedingungen etwas oberhalb der Kondensationstemperatur des dampfförmigen H2O2 liegt (> (100 - 108) °C).

Die Druckschrift DE 30 47 087 A1 offenbart eine Vorrichtung und ein Verfahren der gattungsgemäßen Art, mit denen mittels eines H2O2-Aerosols Verpackungsmaterial, insbesondere von aus Zuschnitten geformten Behältern, entkeimt wird. Dabei wird das flüssige Sterilisierungsmittel, das H2O2, durch Ultraschallvernebelung in einem mit Luft beaufschlagten Nebelraum zerstäubt, der ein festes Volumen aufweist und unter konstantem Druck durch Beaufschlagung mit der Luft aus einer Druckquelle steht. Der Nebelraum oder letzterer und ein mit diesem verbundener Speicherbehälter sind mit einer Abgabeeinrichtung für das Aerosol, bestehend aus dem flüssigen H2O2 und der Luft, verbunden. Der beim Öffnen der Abgabeeinrichtung im Nebelraum oder im Nebelraum und dem Sammelbehälter entstehende Druckverlust wird nach der Schließung der Abgabeeinrichtung durch die Druckquelle wieder kompensiert, wobei die Ultraschallvernebelung für das erforderliche Mengenverhältnis zwischen der dispersen Phase, dem flüssigen H2O2, und der kontinuierlichen Phase, der Luft, sorgt. Das dergestalt erzeugte Aerosol kann taktweise über die Abgabeeinrichtung ausgebracht werden.

Diese bekannte Vorrichtung erzeugt ein mehr oder weniger homogenes Aerosol, wobei die Tröpfchengröße des Aerosols in Abhängigkeit von der angelegten Ultraschallfrequenz gesteuert wird. Die Veränderung der Tröpfchengröße bedeutet allerdings nicht, dass dadurch gleichzeitig auch das Mengenverhältnis zwischen Luft und H2O2 definiert eingestellt werden kann. Ein sterilisierendes Gasgemisch, bestehend aus Luft und dampfförmigem H2O2, wobei letzteres in der vorstehend beschriebenen Weise auf den zu sterilisierenden Oberflächen kondensiert und dabei einen geschlossenen Flüssigkeitsfilm erzeugt, kann mit der bekannten Vorrichtung nicht erzeugt werden, da eine Beheizung nicht vorgesehen ist. Darüber hinaus kann ein über die Zeitdauer der Abgabe des Aerosols näherungsweise konstanter Volumenstrom des Aerosols auf das Verpackungsmaterial nur dann über die Abgabeeinrichtung ausgebracht werden, wenn diese an den Sammelbehälter angeschlossen ist und letzterer einen erheblich größeres Volumen als der Nebelraum aufweist, in dem das Aerosol erzeugt wird.

In der DE 38 24 923 A1 ist eine Vorrichtung zum Sterilisieren von Behältern beschrieben, die einen vorgeschalteten Behälter mit definiertem Volumen und eine darin angeordnete Mehrstoffdüse für Luft und Wasserstoffperoxid aufweist. Das dort gebildete Aerosol wird anschließend über ein am höchsten Punkt des Behälters anschließendes, im weiteren Verlauf senkrecht nach unten orientiertes Rohr, welches außenseits über eine Heizeinrichtung beheizbar ist, abgeleitet und seiner Zweckbestimmung zugeführt.

Die Druckschrift DE 102 06 174 B4 beschreibt ein Verfahren zur Herstellung von verdampftem Wasserstoffperoxid, das bei einer Verpackungsmaschine zum Sterilisieren der Verpackung oder des Verpackungsbehälters bestimmt ist. Dieses Verfahren zeichnet sich dadurch aus, dass zerstäubtes Wasserstoffperoxid und Luft durch eine Leitung geleitet werden, welche mindestens eine wendelförmige Röhre enthält, die sich innerhalb eines Wärmetauschers erstreckt, dass Heißdampf unter Druck durch den Wärmetauscher geleitet wird und dass das Verfahren so gesteuert wird, dass sich das zerstäubte Wasserstoffperoxid und die Luft in der Leitung erhitzen und an einem Abführungsende der Leitung als Dampf austreten.

Die Vorrichtung zur Durchführung des bekannten Verfahrens gemäß DE 102 06 174 B4**,** ebenso wie die Vorrichtung gemäß DE 38 24 923 A1**,** lassen nicht erkennen, ob jeweils Mittel vorgesehen sind, wie die Herstellung des sterilisierenden Gasgemischs bedarfsgerecht gesteuert, die Konzentration, d.h. das Mengenverhältnis zwischen Luft und H2O2 definiert eingestellt und dieses Mengenverhältnis zur Sicherstellung einer hinreichenden Sterilisierung bzw. Desinfizierung laufend und zeitnah überwacht werden kann.

Eine Vorrichtung zum Erzeugen eines aus einem gasförmigen Bestandteil, insbesondere Luft wie Sterilluft, und einem flüssigen Bestandteil, insbesondere einem Sterilisationsmittel wie Wasserstoffperoxid, bestehenden Aerosols ist aus der DE 298 21 687 U1 bekannt. Diese Vorrichtung weist u.a. einen Behälter auf, in dem fortlaufend der flüssige Bestandteil zerstäubt und in einen den Behälter passierenden Gasstrom eingemischt wird, wobei im unteren Behälterbereich eine axial aufwärtsgerichtete Ringdüse für die Bildung eines ringförmigen Gasstroms und inmitten der Ringdüse eine Zerstäuberdüse für den flüssigen Bestandteil des Aerosols angeordnet ist. In eine an das obere Ende des Behälters angeschlossene Abförderleitung für das Aerosol kann ein Heizaggregat eingeschaltet sein, um sicherzustellen, dass das Aerosol bei Erreichen seines Einsatzortes, beispielsweise eine Flaschensterilisationsstation, die erwünschte Temperatur aufweist.

Da per Definition ein Aerosol ein disperses System darstellt, in dem die kontinuierliche Phase das Gas, im vorliegenden Fall die Luft bzw. Sterilluft, bildet und die disperse Phase flüssig oder fest ist, im vorliegenden Fall flüssiges H2O2 in Form feiner und feinster Tröpfchen, könnte durch die Verwendung des Begriffs "Aerosol", wie vorstehend zitiert, der Eindruck entstehen, dass eine hinreichende sterilisierende bzw. desinfizierende Wirkung eines Gemischs aus Luft und H2O2 auch in Form eines Aerosols gemäß der vorg. Definition erreicht werden kann oder soll. Dass dies nicht zutrifft und auch nicht beabsichtigt ist und der Begriff "Aerosol" im obigen Zitat vermutlich in Unkenntnis seiner wahren Definition verwendet wird, ergibt sich aus einer Feststellung an einleitender Stelle in der DE 298 21 687 U1**,** die da lautet, dass nämlich die Vorrichtung nach der Erfindung bei hoher Leistung ein besonders homogenes, tröpfchenfreies Aerosol erzeugt, das bei Bestehen aus beispielsweise Sterilluft und Wasserstoffperoxid eine hohe Sterilisations- bzw. Desinfektionskraft aufweist. Ein homogenes, tröpfchenfreies Aerosol ist zweifelsfrei als ein Gemisch aus Sterilluft und verdampften Wasserstoffperoxid-Tröpfchen zu deuten, denn bei Abwesenheit dieser Tröpfen, ohne dass diese durch Beheizung in Dampfform überführt worden wären, gäbe es kein Gemisch aus Sterilluft und Wasserstoffperoxid-Dampf, sondern nur erhitze Sterilluft, die für sich gesehen keine Sterilisations- bzw. Desinfektionskraft aufweist.

Auch diese bekannte Vorrichtung zum Erzeugen eines Aerosols lässt nicht erkennen, ob Mittel vorgesehen sind, wie die Herstellung des sterilisierenden Gasgemischs bedarfsgerecht gesteuert, die Konzentration, d.h. das Mengenverhältnis zwischen Luft und H2O2 definiert eingestellt und das vorg. Mengenverhältnis zur Sicherstellung einer hinreichenden Sterilisierung bzw. Desinfizierung laufend und zeitnah überwacht werden kann.

Aus der DE 35 40 161 A1 ist ein Verfahren zum Entkeimen von Verpackungsmaterial, insbesondere von Verpackungsbehältern, mittels eines Wasserstoffperoxid enthaltenden flüssigen Entkeimungsmittels bekannt, bei dem das Entkeimungsmittel zerstäubt und mit Druckluft gemischt, das entstehende Gemisch verdampft und danach das Dampf-Luft-Entkeimungsmittel-Gemisch in Turbulenz versetzt und auf die zu entkeimende Fläche des Verpackungsmaterials bzw. -behälters aufgeblasen und dort der Dampf zum Kondensieren gebracht wird. Dabei wird dem Gemisch zunächst eine Rotationsbewegung entlang einer Mittelachse aufgezwungen und danach eine durch die Zentrifugalwirkung der Rotationsströmung erzeugte Grenzschicht der Gemischflüssigkeit zumindest teilweise in Gegenrichtung zur Hauptströmungsrichtung des Gemischs zwangsläufig geführt.

Die Vorrichtung zur Durchführung des Verfahrens weist u.a. einen Vorratsbehälter für flüssiges, Wasserstoffperoxid enthaltendes Entkeimungsmittel, eine Zerstäuber-Blas-Vorrichtung und eine dieser nachgeschaltete Verdampfungseinrichtung auf. Letztere besteht aus einem druckluftbeaufschlagten Sprührohr mit als Spiralfeder und Leitelement ausgebildeten Einbauten zur Strömungsführung des Dampf-Luft-Entkeimungsmittel-Gemischs. Dabei sind das Leitelement von wenigstens einem im Sprührohr eingesetzten Drallkörper und die Spiralfeder von wenigstens einem, in Strömungsrichtung gesehen, hinter dem Drallkörper im Sprührohr befindlichen, eine zur Drallnut des Drallkörpers gegensinnig verlaufende Steigung aufweisenden Einsatz gebildet.

Die US-A-2 242 466 offenbart einen sich nach unten verjüngenden konischen Behälter eines Verdampferbehälters mit einem doppelwandig ausgeführten Behältermantel, wobei ein von den beiden Wandungen gebildeter Ringraum von einer Heizflüssigkeit durchströmt wird.

Das Dokument US-A-4 512 935 beschreibt ein nach unten offenes Verdampferrohr, an dessen oberer stirnseitiger Begrenzung eine Zweistoffdüse angeordnet ist. Im Innern des Verdampferrohres ist über dessen axiale Erstreckungslänge eine wendelförmige Leitschiene angeordnet, die ein unten in das Verdampferrohr tangential eintretendes und dieses oben tangential verlassendes heißes Gas in seiner aufwärts gerichteten Rotationsströmung unterstützt. Das heiße Gas wird dadurch im Gegenstrom zum dem aus der Zweistoffdüse austretenden, das Verdampferrohr von oben nach unten durchsetzenden und dieses am unteren Ende verlassenden Flüssigkeits-Gas-Gemisch geführt.

Aus der US-A-4 537 749 ist eine Vorrichtung zur Sterilisierung von oben offenen Behältnissen bekannt. Zu diesem Zweck wird eine Kappe auf den oberen Rand des Behältnisses aufgesetzt und eine im Kopfraum der Kappe angeordnete Zweistoffdüse zerstäubt ein Gemisch aus Druckluft und einem flüssigen Sterilisierungsmittel, vorzugsweise Wasserstoffperoxid, in das jeweilige Behältnis. Eine sich an der Zweistoffdüse mittelbar oder unmittelbar abstützende und sich unterhalb derselben erstreckende, konusförmig ausgebildete elektrische Heizwendel greift in den von der Zweistoffdüse ausgebrachten Strahl des Gemischs ein, erhitzt dieses und verdampft dabei das flüssige Sterilisierungsmittel.

Die WO 98/34649 A offenbart ein Verfahren zum Verdampfen und Überhitzen eines Sterilisierungsmittels, bei dem unter anderem ein gasförmiges Trägermedium, vorzugsweise Heißluft, in den Sprühstrahl des Sterilisierungsmittels, vorzugsweise Wasserstoffperoxid, eingeführt wird und dieses gasförmige Trägermedium eine Temperatur im Bereich von 130 °C bis 170 °C hat. Die Überhitzung des Gemischs aus Heißluft und Wasserstoffperoxid erfolgt in einem elektrisch beheizten Heizkörper, dessen Temperatur über einen Temperaturfühler abgefühlt und gesteuert wird.

Aufgabe der vorliegenden Erfindung ist es, eine Vorrichtung und ein Verfahren zum Erzeugen eines sterilisierenden Gasgemischs, insbesondere bestehend aus Luft bzw. Sterilluft und Wasserstoffperoxid in Dampfform, anzugeben, die eine bedarfsorientierte Erzeugung des Gasgemischs und eine definierte Einstellung des Mengenverhältnisses der beiden das Gasgemisch bildenden Bestandteile sicherstellen und die darüber hinaus die Möglichkeit bieten, eine zeitnahe Überwachung dieses Mengenverhältnisses durchzuführen.

### ZUSAMMENFASSUNG DER ERFINDUNG

Diese Aufgabe wird durch eine Vorrichtung zum Erzeugen eines sterilisierendenGasgemischs mit den Merkmalen des Anspruchs 1 und durch ein diesbezügliches Verfahren unter Verwendung einer Vorrichtung gemäß Anspruch 1 mit den Merkmalen des Nebenanspruchs 19 gelöst. Bevorzugte Ausführungsbeispiele sind in den jeweils abhängigen Ansprüchen angegeben. In Anspruch 27 wird eine bevorzugte Verwendung der Vorrichtung angegeben.

Die Vorrichtung zeichnet sich dadurch aus, dass ein Behälter in seinem oberen Teil aus dem Zerstäuber- und Speicherbehälter besteht, in dem zum einen die Zerstäubung des flüssigen zweiten Bestandteils (z.B. flüssiges H2O2) und gleichzeitig die Vermischung mit dem gasförmigen ersten Bestandteil (z.B. Luft oder Sterilluft) erfolgt und der zum anderen eine Speicherfunktion für das erzeugte Aerosol wahrnimmt. Das feste Speichervolumen ist dabei um ein Vielfaches (beispielsweise 20 bis 30fach) größer als das jeweilige Volumen, das beispielsweise in der Verpackungsmaschine im Zuge der intermittierenden Abgabe des sterilisierenden Gasgemischs im jeweiligen Abgabe- bzw. Dosiertakt über die Abgabeeinrichtung (beispielsweise ein Dosierventil) ausgebracht wird. Der Zerstäuber- und Speicherbehälter weist wenigstens eine gesteuerte Zerstäuberdüse auf, an die die beiden auszubringenden Bestandteile getrennt voneinander herangeführt werden und über die bei Ansteuerung sowohl der gasförmige erste Bestandteil als auch der flüssige zweite Bestandteil im vorgegebenen festen Mengenverhältnis in das im Zerstäuber- und Speicherbehälter bereits vorhandene Aerosol eingedüst werden.

Der Zerstäuber- und Speicherbehälter geht unterhalb an seinem Abführungsende in einen Verdampferbehälter mit einer Heizeinrichtung über. Dort kann das Aerosol oder ein erzeugtes, bereits einphasiges Fluid aus den beiden Mischungsbestandteilen unmittelbar im Anschluss an seine Erzeugung auf die erforderliche Temperatur indirekt, d.h. rekuperativ beheizt werden, bei der zum einen die Überführung der flüssigen dispersen Phase in Dampfform sichergestellt und/oder eine Rückkehr von bereits vor der Beheizung verdampften Bestandteilen in die flüssige disperse Phase ausgeschlossen ist.

Dem Behälter ist eine Druckregelungs-Einrichtung zugeordnet, die den vorgegebenen konstanten Druck im Behälter aufrecht erhält. Eine Konstanz des Druckes im Behälter bedeutet, dass die Strömungsbedingungen im Rohrleitungs- und Apparatesystem zwischen Behälter und der Abgabeeinrichtung einerseits und dem Behälter und der Quelle für den gasförmigen ersten Bestandteil (z.B. Luft bzw. Sterilluft) andererseits eindeutig definiert sind, so dass jedem Abgabe- bzw. Dosiertakt ein bestimmbares Volumen des aus dem Behälter auf der einen Seite abgeführten sterilisierenden Gasgemischs und des dem Behälter auf der anderen Seite zugeführten gasförmigen ersten Bestandteils zugeordnet werden kann und somit aus einer leicht erfassbaren Anzahl von Abgabe- bzw. Dosiertakten über eine bestimmbare erste Zeitdauer eine Bilanzierung der jeweiligen Gesamtvolumina gegeben ist.

Die Erfindung besteht unter den Prämissen der vorstehend genannten Vorrichtungsmerkmale darin, dass die Abgabeeinrichtung ein Dosierventil umfasst, welches über eine Dosierleitung mit dem Verdampferbehälter verbunden ist.

Immer dann, wenn die Abgabeeinrichtung (Dosierventil) angesteuert und damit sterilisierendes Gasgemisch aus dem Behälter abgeführt wird, erfährt auch die wenigstens eine Zerstäuberdüse eine Ansteuerung. Die wenigstens eine Zerstäuberdüse bringt in einem voreingestellten festen Mengenverhältnis gleichzeitig die beiden Mischungsbestandteile (z.B. Luft und flüssiges H2O2) in den Zerstäuberund Speicherbehälter ein, wobei die Druckregelungs-Einrichtung dafür sorgt, das der Abfluss aus dem und der Zufluss zu dem Behälter unter der Bedingung konstanten Druckes im Behälter stattfindet.

Die Konstanz des Druckes im Behälter schafft auch eindeutige Strömungsbedingungen in der Leitung zwischen der wenigstens einen Zerstäuberdüse und einem Vorratsbehälter für den flüssigen zweiten Bestandteil (z.B. H2O2'), so dass ein einmal voreingestelltes festes Mengenverhältnis zwischen den an der Zerstäuberdüse anstehenden Mischungsbestandteilen erhalten bleibt.

Mit der vorgeschlagenen Vorrichtung sind damit eine bedarfsorientierte Erzeugung des Gasgemischs und eine definierte Einstellung des vorgegebenen festen Mengenverhältnisses der beiden das Gasgemisch bildenden Bestandteile sichergestellt. Im Detail lassen sich einzelne vorgeschlagene Maßnahmen auch in gleichwirkender Weise abgewandelt lösen; wesentlich ist dabei, dass die erfindungsgemäß vorgesehene Konstanz des Druckes des Aerosols im Behälter definierte und reproduzierbare Bedingungen für alle am Prozess des Erzeugens eines sterilisierenden Gasgemischs beteiligten Stoffströme schafft.

Gemäß einem weiteren Vorschlag ist der Verdampferbehälter in Form eines gegenüber dem Zerstäuber- und Speicherbehälter verjüngten rohrförmigen Behälterteils ausgebildet ist. Durch diese Ausgestaltung werden im Verdampferbehälter günstige Strömungsbedingungen für einen Wärmeaustausch geschaffen, da hier die in der Zeiteinheit im Bedarfsfall durchgesetzten Volumina klein gegenüber dem Speichervolumen des Zerstäuber- und Speicherbehälters sind.

Die im Verdampferbehälter angeordnete Heizeinrichtung weist gemäß einem ersten Vorschlag wenigsten ein von außen beheizbares Rohr auf, in dem das zu beheizende Aerosol oder Fluid strömt, wobei der Außenmantel der Heizeinrichtung, der ein Heizmedium innenseits aufnimmt, vom Mantel des Verdampferbehälters gebildet ist. Es ist vorgesehen, dieses wenigstens eine Rohr entweder geradlinig oder, um die Baulänge des Verdampferbehälters nicht zu groß werden zu lassen, gewendelt auszuführen.

Eine besonders vorteilhafte und wirksame Ausgestaltung wird mit einem zweiten Vorschlag geschaffen, bei dem die Heizeinrichtung als Rohrbündel-Wärmeaustauscher ausgebildet ist, in dem das zu beheizende Aerosol oder Fluid auf der Innenseite von wenigstens zwei parallel geschalteten Innenrohren strömt, wobei der Außenmantel des Rohrbündel-Wärmeaustauschers, der ein Heizmedium innenseits aufnimmt, vom Mantel des Verdampferbehälters gebildet ist. Diesbezüglich ist weiterhin vorgesehen, dass sich der Rohrbündel-Wärmeaustauscher über die gesamte Länge des Verdampferbehälters erstreckt.

Eine besonders wirksame Beheizung des Aerosols bei einer eindeutig bestimmbaren Temperatur stellt ein anderer Vorschlag sicher, der vorsieht, dass die Heizeinrichtung dampfbeheizt ist, vorzugsweise mit Wasserdampf unter Sättigungsbedingungen. Der kondensierende Wasserdampf stellt einerseits seine Kondensationswärme an den Wärmeaustauscherflächen zur Verfügung und die primärseitige Heiztemperatur ist über den Dampfdruck exakt einstell- und regelbar und sie bleibt über den gesamten Strömungsweg des beheizten Aerosols konstant.

Es hat sich als vorteilhaft hinsichtlich einer guten Durchmischung erwiesen, wenn im Mantel des Zerstäuber- und Speicherbehälter zwei Zerstäuberdüsen angeordnet sind. Die Wirksamkeit der Ein- und Durchmischung der über die Zerstäuberdüsen in den Behälter eingebrachten beiden Mischungsbestandteile wird verbessert, wenn die Zerstäuberdüsen, wie dies auch vorgeschlagen wird, dabei diametral zueinander angeordnet sind. Eine weitere Verbesserung wird erreicht, wenn die Zerstäuberdüsen zusätzlich auch in der Höhe axial versetzt zueinander angeordnet sind.

Besonders vorteilhaft ist es, die Zerstäuberdüsen als Zweistoffdüsen auszubilden. Diesen Zweistoffdüsen werden die beiden auszubringenden Bestandteile (z.B. Luft bzw. Sterilluft und flüssiges H2O2') getrennt zugeführt, und bei Ansteuerung der Zweistoffdüse werden die beiden Bestandteile in einem vorgegebenen festen Mengenverhältnis gemischt und dabei in den Zerstäuber- und Speicherbehälter ausgedüst. Dieses vorgegebene feste Mengenverhältnis ist nach einem weiteren Vorschlag durch eine veränderbare Größe und/oder eine veränderbare Zeitdauer eines Öffnungshubes eines Schließgliedes der Zerstäuberdüse einstellbar.

Da der gasförmige erste Bestandteil meist Luft in Form von Druckluft ist, die nach dem Verlassen einer Druckluftquelle speziell aufbereitet und vorgeheizt wird, vereinfacht es die Vorrichtung, wenn, wie dies auch vorgeschlagen wird, die Zerstäuberdüsen mit Druckluft aus der vorg. Druckluftquelle pneumatisch angesteuert werden.

Um eine zeitnahe Überwachung des Mengenverhältnisses der beiden das Aerosol bildenden Bestandteile durchzuführen, sieht eine weitere Ausführungsform der Vorrichtung gemäß der Erfindung vor, dass in jeder Leitung, die als Zuleitung zu der(den) Zerstäuberdüse(n) für den flüssigen Bestandteil fungiert, ein Durchflussmesser angeordnet ist, über den die in einer vorbestimmten ersten Zeitdauer gemessen Volumenströme bzw. Volumina des flüssigen zweiten Bestandteils erfasst und registriert werden. Darüber hinaus sind diese Durchflussmesser so ausgestattet, dass sie Steuersignale für einen Überwachungsprozess generieren können. Da der in der ersten Zeitdauer in den Behälter eingebrachte Volumenstrom bzw. das daraus resultierende Volumen des gasförmigen ersten Bestandteils, wie oben angegeben, ermittelt und damit bekannt ist, kann mit den insgesamt zur Verfügung stehenden diesbezüglichen Informationen jederzeit eine Bilanzierung der Stoffströme und damit eine Überwachung des vorgegebenen festen Mengenverhältnisses der das Aerosol bildenden Bestandteile vorgenommen werden, so dass die Qualität der Sterilisation bzw. Desinfektion in der Verpackungsmaschine laufend und zeitnah kontrollierbar ist.

Eine weitere Kontrollmöglichkeit des besagten vorgegebenen festen Mengenverhältnisses in bestimmbaren Zeitabständen ergibt sich, wenn die Zeitdauer für den Verbrauch einer vorgegebenen Menge des flüssigen zweiten Bestandteils (z.B. flüssiges H2O2') erfasst und diese Menge mit der in diesem Zeitraum ausgebrachten Menge des gasförmigen ersten Bestandteils (z.B. Luft bzw. Sterilluft) ins Verhältnis gesetzt wird. Darüber hinaus kann der Verbrauch einer vorgegebenen Menge des flüssigen zweiten Bestandteils zur Kontrolle der Messungen an den Durchflussmessern verwendet werden. Um die vorg. Kontrollmöglichkeit zu schaffen, wird vorgeschlagen, dass die Zerstäuberdüse(n) über die Leitung(en) mit einem in seinem Füllstand geregelten Vorratsbehälter für den flüssigen Bestandteil verbunden ist(sind). Außerdem ist weiterhin vorgesehen, dass eine dem Vorratsbehälter zugeordnete Füllstandsänderung Steuersignale generiert.

Um bereits im Speicherbehälter beim Eindüsen des flüssigen zweiten Bestandteils eine teilweise oder vollständige Verdampfung desselben zu erreichen, sieht eine Ausgestaltung der Vorrichtung vor, dass in einer für die Zufuhr des gasförmigen ersten Bestandteils in den Speicherbehälter vorgesehenen ersten Leitung ein Vorwärmer angeordnet ist.

Unter Verwendung einer Vorrichtung gemäß Anspruch 1 besteht der verfahrenstechnische Lösungsgedanke der Erfindung zunächst darin, dass das Aerosol in dem festen Speichervolumen bei dem dort vorgegebenen und durch Druckregelung konstant gehaltenen Druck bereitgestellt wird. Darüber hinaus initiiert die bedarfsorientierte gesteuerte Abfuhr des Aerosols aus dem festen Speichervolumen eine zeitnahe Zufuhr des gasförmigen ersten Bestandteils und des flüssigen zweiten Bestandteils in einem vorgegebenen festen Mengenverhältnis in dieses Speichervolumen. Zufuhr und Abfuhr werden dabei, damit der Druck im Behälter konstant bleibt, zeitnah ausgeglichen. Die Zufuhr wird dabei in Abhängigkeit von der Taktfrequenz und der Zeitdauer der intermittierenden Abfuhr gesteuert.

Die verfahrenstechnische Lösung beinhaltet weiterhin, dass das Aerosol zum Verdampfen des flüssigen zweiten Bestandteils oder das gewonnene gasförmige Fluid jeweils anschließend auf seinem weiteren Strömungsweg indirekt, d.h. rekuperativ beheizt und das schließlich gewonnene Gasgemisch, gebildet aus dem gasförmigen ersten Bestandteil und dem dampfförmigen zweiten Bestandteil, seiner Zweckbestimmung zugeführt wird. Ein gasförmiges Fluid liegt bereits im Zerstäuber- und Speicherbehälter dann vor, wenn, wie nachfolgend noch dargestellt, der gasförmige erste Bestandteil vor der Gemischbildung hinreichend vorgewärmt wird. Die Ausbringung des Gasgemischs anstelle des vielfach im Stand der Technik üblichen Aerosols hat den entscheidenden Vorteil, dass durch das Kondensieren des dampfförmigen zweiten Bestandteils auf den zu sterilisierenden Oberflächen in jedem Falle ein geschlossener, sterilisierender Flüssigkeitsfilm sichergestellt ist.

Eine besonders wirksame Beheizung des Aerosols bei einer eindeutig bestimmbaren Temperatur stellt ein Vorschlag sicher, der vorsieht, dass das Aerosol mittels Heißdampf, insbesondere Wasserdampf im Sättigungszustand, beheizt wird. Die Vorteile einer diesbezüglichen Behandlung sind vorstehend bereits beschrieben. Dabei erfolgt nach einer bevorzugten Ausgestaltung des Verfahrens die rekuperative Beheizung mit einer Oberflächentemperatur von maximal 105 °C.

Um bereits im Speicherbehälter beim Eindüsen des flüssigen zweiten Bestandteils eine teilweise oder vollständige Verdampfung desselben zu erreichen, sieht eine weitere Ausgestaltung des Verfahrens vor, dass der gasförmige erste Bestandteil vor der Beimengung des flüssigen zweiten Bestandteils vorgewärmt wird. Hier wird vorgeschlagen, diesen vorzugsweise auf eine Temperatur ϑ₁ = ϑ_{AR} (ϑ_{As}) vorzuwärmen, die über der späteren Temperatur ϑ₃ = ϑ_{G} des sterilisierfähigen Gasgemischs liegt, bevorzugt auf ϑ₁ = 130 °C.

Um die aufgabengemäß herausgestellte definierte Einstellung des vorgegebenen festen Mengenverhältnisses der beiden das Gasgemisch bildenden Bestandteile zu erleichtern und zu kontrollieren, sieht ein anderer Vorschlag vor, dass der am Ort der Verwendung des Gasgemischs ausgebrachte Istwert des Mengenverhältnis messtechnisch ermittelt und dieser Messwert zur Voreinstellung der dem festen Speichervolumen zugeführten Volumenströme des gasförmigen ersten Bestandteils und des flüssigen zweiten Bestandteils verwendet wird.

Mit einer anderen vorteilhaften Ausgestaltung des Verfahrens gemäß der Erfindung, die eine Bilanzierung der Stoffströme ermöglicht, wird eine laufende und zeitnahe Überwachung und Kontrolle des realisierten vorgegebenen festen Mengenverhältnisses verwirklicht. Hierzu ist vorgesehen, dass über eine vorbestimmte erste Zeitdauer der Verwendung des Gasgemischs der Volumenstrom des gasförmigen ersten Bestandteils ermittelt und der Volumenstrom des flüssigen zweiten Bestandteils gemessen werden, dass aus diesen Werten der Istwert des Mengenverhältnis berechnet wird, und dass bei einer Abweichung vom vorgegebenen festen Mengenverhältnis (Sollwert) um ein vorgegebenes Toleranzintervall ein Alarm- und/oder Abschaltsignal generiert wird.

Eine weitere Kontrollmöglichkeit in bestimmbaren Zeitabständen ergibt sich nach einer weiteren Ausgestaltung des Verfahrens dadurch, dass für eine vorgegebene Änderung des Füllstandes in einem Vorratsbehälter für den flüssigen zweiten Bestandteil eine hierfür notwendige zweite Zeitdauer gemessen wird und letztere zur Kontrolle des gemessenen Volumenstromes des flüssigen zweiten Bestandteils und/oder des vorgegebenen festen Mengenverhältnisses (Sollwert) herangezogen wird.

Die vorstehend beschriebene Vorrichtung eignet sich insbesondere zur Verwendung bei einer Verpackungsmaschine zum Sterilisieren des Verpackungsmittels oder der durch Behälterwände begrenzten Verpackungszone.

### KURZBESCHREIBUNG DER ZEICHNUNGEN

Die Erfindung wird nachfolgend anhand eines bevorzugten Ausführungsbeispiels näher erläutert. Während die Erfindung in den verschiedensten Ausbildungsformen realisierbar ist, werden in den Zeichnungen das besonders bevorzugte Ausführungsbeispiel gezeigt und nachfolgend beschrieben unter der Voraussetzung, dass dieses nur ein Beispiel für die Erfindung darstellt, nicht aber die Erfindung auf dieses speziell dargestellte Beispiel beschränkt ist. Es zeigen
- **Figur 1**: die Vorrichtung gemäß der Erfindung in schematischer, symbolhafter Darstellung in Anlehnung an ein Rohrleitungs- und Instrumentenfließbild (RI-Fließbild);
- **Figur 2**: in einer halb Schnitt- (links) und halb Ansicht-Darstellung (rechts) eine bevorzugte Ausführungsform eines innerhalb der Vorrichtung gemäß **Figur 1** angeordneten Behälters zum Erzeugen eines sterilisierenden Gasgemischs und
- **Figur 2a**: in perspektivischer Darstellung den Behälter gemäß **Figur 2****,** wobei dieser sektoriell über seine gesamte Länge aufgeschnitten dargestellt ist.

### DETAILLIERTE BESCHREIBUNG

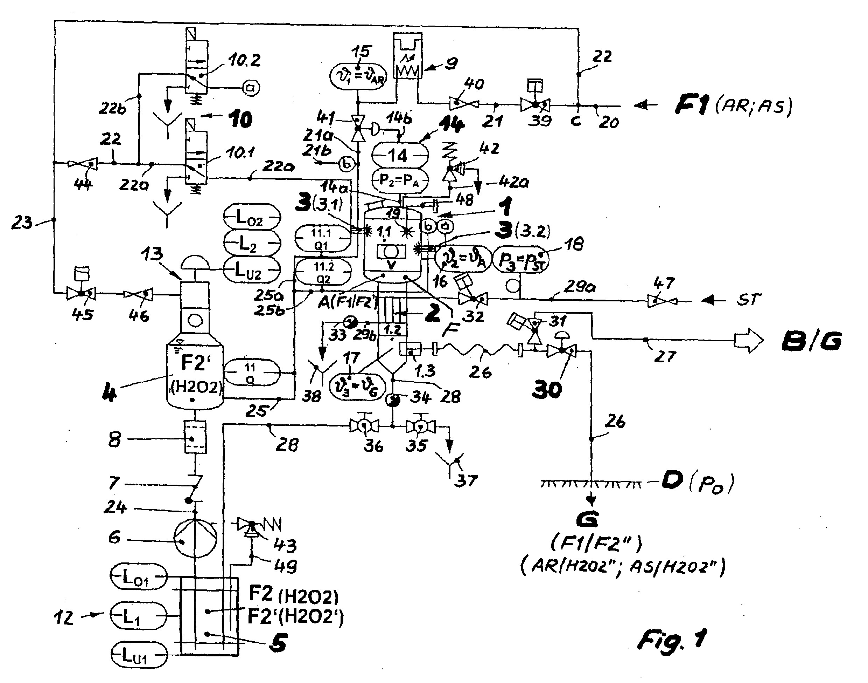

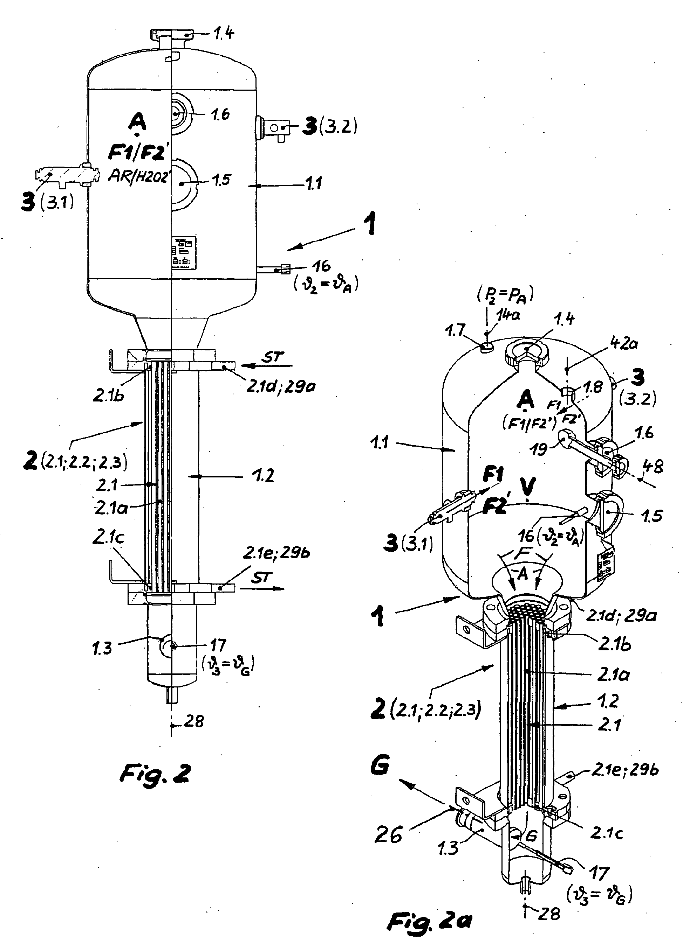

Die zentrale Komponente der erfindungsgemäßen Vorrichtung bildet ein Behälter 1 mit einem festen Speichervolumen V **(****Figur 1****;** **Figuren 2, 2a****),** der in seinem oberen Teil aus einem Zerstäuber- und Speicherbehälter 1.1 besteht. Letzterer geht unterhalb an seinem Abführungsende für ein Aerosol A in einen Verdampferbehälter 1.2 über, der in Form eines gegenüber dem Zerstäuber- und Speicherbehälter 1.1 verjüngten rohrförmigen Behälterteils ausgebildet ist. Das Aerosol A als sog. disperses System besteht im allgemeinsten Fall aus einem gasförmigen ersten Bestandteil F1 als kontinuierlicher Phase (z.B. Luft AR oder Sterilluft AS) und einem flüssigen zweiten Bestandteil F2' als disperser Phase (z.B. flüssiges H2O2'). Der zweite Bestandteil F2 (z.B. Wasserstoffperoxid, abgekürzt H₂O₂, vereinfacht als H2O2 geschrieben) nimmt im Aerosol A seine flüssige Phase F2' ein. Dieser flüssige zweite Bestandteil F2' wird beim Durchlaufen einer im Verdampferbehälter 1.2 angeordneten Heizeinrichtung 2 verdampft, so dass der Bestandteil nunmehr nach dieser Zustandsänderung als dampfförmiger zweiter Bestandteil F2" (z.B. H2O2") zusammen mit dem gasförmigen ersten Bestandteil F1 (z.B. AR oder AS) ein sterilisierendes Gasgemisch G bildet, das den Verdampferbehälter 1.2 über einen Austrittsstutzen 1.3 verlässt und über eine Dosierleitung 26 auf dem Weg über eine Abgabeeinrichtung (Dosierventil) 30 einer Dosierstelle D, beispielsweise in einer nicht dargestellten Verpackungsmaschine, zugeführt wird.

Im Zerstäuber- und Speicherbehälter 1.1 ist im unteren Bereich eine zweite Temperatur-Messeinrichtung 16 angeordnet, mit der eine zweite Temperatur ϑ₂ = ϑ_{A} des erzeugten Aerosols A gemessen wird. In der Nähe des Austrittsstutzens 1.3 befindet sich im Mantel des Verdampferbehälters 1.2 eine dritte Temperatur-Messeinrichtung 17, mit der eine dritte Temperatur ϑ₃ = ϑ_{G} des abströmenden sterilisierenden Gasgemischs G gemessen wird. Vor der Abgabeeinrichtung 30, in Strömungsrichtung des Gasgemischs G gesehen, zweigt von der Dosierleitung 26 eine Umgehungsleitung 27 ab, die über ein erstes Absperrventil 31 absperrbar ist. Über diese Umgehungsleitung 27 kann Abluft B, die beim Anfahren des Behälters 1 anfällt, oder sterilisierendes Gasgemisch G, das über die Abgabeeinrichtung 30 nicht ausgebracht werden kann oder darf, in einen nicht dargestellten Abluftkanal abgeführt werden.

Die Heizeinrichtung 2, vorzugsweise als Rohrbündel-Wärmeaustauscher 2.1 **(****Figuren 2, 2a****)** ausgebildet, ist bevorzugt dampfbeheizt, mit Heißdampf, vorzugsweise als Wasserdampf ST im Sättigungszustand, wobei der Wasserdampf ST über eine Zulaufleitung 29a auf dem Weg über eine vierte Drosseleinrichtung 47 und ein zweites Absperrventil 32 zu einem Zulaufstutzen 2.1d in einer oberen Rohrträgerplatte 2.1 b des Rohrbündel-Wärmeaustauschers 2.1 gelangt und von dort in dessen Außenmantel einströmt, der vom Mantel des Verdampferbehälters 1.2 gebildet ist. Der Rohrbündel-Wärmeaustauscher 2.1 erstreckt sich nahezu über die gesamte Länge des Verdampferbehälters 1.2. Der Wasserdampf ST kondensiert dabei an den Außenwänden einer Vielzahl von Innenrohren 2.1 a, auf deren jeweiliger Innenseite das zu beheizende Aerosol A strömt. Vor dem zweiten Absperrventil 32 ist in der Zulaufleitung 29a eine Druck-Messeinrichtung 18 angeordnet, mit der ein Druck p₃ = p_{ST} des Heißdampfes ST gemessen wird. Das Kondensat des Heißdampfes ST verlässt den Rohrbündel-Wärmeaustauscher 2.1 über einen Ablaufstutzen 2.1e an einer unteren Rohrträgerplatte 2.1c und gelangt über eine Ablaufleitung 29b, in der ein erster Kondensatabscheider 33 angeordnet ist, in einen zweiten Ablauf 38.

Anstelle des vorstehend beschriebenen Rohrbündel-Wärmeaustauschers 2.1 kann die Heizeinrichtung 2 auch in Form wenigstens eines von außen beheizbaren Rohres 2.2, 2.3 ausgebildet sein, in dem das zu beheizende Aerosol A strömt, wobei der Außenmantel der Heizeinrichtung 2, der das Heizmedium innenseits aufnimmt, vom Mantel des Verdampferbehälters 1.2 gebildet ist. Dabei ist das wenigstens eine Rohr als geradliniges Rohr 2.2 oder als gewendeltes Rohr 2.3 ausgeführt.

An seinem unteren Ende ist an den Verdampferbehälter 1.2 eine erste Rückführleitung 28 angeschlossen, über die der ggf. aus dem Gasgemisch G auskondensierende flüssige zweite Bestandteil F2' (z.B. flüssiges H2O2') auf dem Weg über einen zweiten Kondensatabscheider 34 an einer Verzweigungsstelle entweder einerseits über einen ersten Ablauf 37, der über ein drittes Absperrventil 35 absperrbar ist, verworfen oder andererseits in einen Bereitstellungsbehälter 5 rückgeführt wird, wobei dieser Abschnitt der ersten Rückführleitung 28 über ein viertes Absperrventil 36 absperrbar ist.

Der Bereitstellungsbehälter 5 nimmt den zweiten Bestandteil F2 (z.B. H2O2) in dessen flüssiger Phase F2' (H2O2') auf und ist in seinem Füllstand überwacht, wobei hierfür eine Füllstandsanzeige-Einrichtung 12 vorgesehen ist, die dort zwischen einem maximalen ersten Füllstand Lₒ₁ (oberer Grenzwert) und einem minimalen ersten Füllstand Lᵤ₁ (unterer Grenzwert) einen ersten Füllstand L₁ ermittelt und bei Erreichen des jeweiligen Grenzwertes Lₒ₁, Lᵤ₁ ein Schaltsignal generiert.

Der flüssige zweite Bestandteil F2' (z.B. flüssiges H2O2') wird mittels einer in einer Füllleitung 24 angeordneten Fördereinrichtung 6, vorzugsweise eine Dosierpumpe relativ kleiner Förderleistung, auf dem Weg über ein Rückschlagventil 7 und ein Sieb 8 einem Vorratsbehälter 4, vorzugsweise von unten, zugeführt. Der Vorratsbehälter 4 ist mit einer Füllstandsregelungs-Einrichtung 13 versehen, die dort zwischen einem maximalen zweiten Füllstand Lₒ₂ (oberer Grenzwert) und einem minimalen zweiten Füllstand Lᵤ₂ (unterer Grenzwert) einen zweiten Füllstand L₂ regelt. Die Füllstandsregelungs-Einrichtung 13 ist darüber hinaus in der Lage, für eine vorgegebene Füllstandsänderung im Vorratsbehälter 4 eine hierfür notwendige zweite Zeitdauer Δt_{F} zu ermitteln, die für Bilanzierungs- und Kontrollzwecke Verwendung findet. Die Druckseite der Fördereinrichtung 6 ist über eine zweite Rückführleitung 49, die über ein zweites Sicherheitsventil 43 abgesichert ist, mit dem Bereitstellungsbehälter 5 verbunden.

Der flüssige zweite Bestandteil F2' (z.B. flüssiges H2O2') gelangt über eine vierte Leitung 25, die sich in eine fünfte Verzweigungsleitung 25a und eine sechste Verzweigungsleitung 25b verzweigt, zu einer gesteuerten ersten Zerstäuberdüse 3.1 bzw. einer gesteuerten zweiten Zerstäuberdüse 3.2, die im Mantel des Zerstäuber- und Speicherbehälters 1.1, vorzugsweise diametral zueinander und in der Höhe axial zueinander versetzt, angeordnet sind. Die Zerstäuberdüsen 3.1, 3.2 werden bevorzugt als Zweistoffdüsen ausgebildet, die jeweils einen Anschluss zur Zufuhr des gasförmigen Bestandteils F1 (z.B. Luft AR oder Sterilluft AS) und einen Anschluss zur Zufuhr des flüssigen Bestandteils F2' (z.B. flüssiges H2O2') besitzen. Die Anordnung von zwei Zerstäuberdüsen 3.1, 3.2 ist nicht zwingend; es ist wenigstens eine Zerstäuberdüse 3 vorzusehen, aber es können auch mehr als zwei angeordnet werden. In der vierten Leitung 25 ist ein Durchflussmesser 11, in der fünften Verzweigungsleitung 25a ist ein erster Durchflussmesser 11.1 und in der sechsten Verzweigungsleitung 25b ist ein zweiter Durchflussmesser 11.2 angeordnet. Mit dem Durchflussmesser 11 wird ein gesamter Volumenstrom Q, der in Summe für die beiden Zerstäuberdüsen 3.1, 3.2 erforderlich ist, gemessen, während der erste Durchflussmesser 11.1 allein einen ersten Teilvolumenstrom Q₁ zur ersten Zerstäuberdüse 3.1 und der zweite Durchflussmesser 11.2 allein einen zweiten Teilvolumenstrom Q₂ zur zweiten Zerstäuberdüse 3.2 messen.

Die Ansteuerung der Zerstäuberdüsen 3.1, 3.2 erfolgt vorzugsweise pneumatisch. Im Ausführungsbeispiel ist der ersten Zerstäuberdüse 3.1 ein erstes Steuerventil 10.1 und der zweiten Zerstäuberdüse 3.2 ein zweites Steuerventil 10.2 zugeordnet. Grundsätzlich gelingt die Erzeugung des Aerosols A im Zerstäuber- und Speicherbehälter 1.1 auch mit der einzigen Zerstäuberdüse 3, die dann über ein Steuerventil 10 angesteuert wird. Das Druckmittel zur Ansteuerung der Steuerventile 10 bzw. 10.1, 10.2, Druckluft AR, wird über eine Zuführleitung 20 aus einer nicht dargestellten Druckluftquelle bereitgestellt, die auch den gasförmigen ersten Bestandteil F1 (z.B. Luft AR oder Sterilluft AS) liefern kann, und gelangt an einer Verzweigungsstelle c über eine zweite Leitung 22 zu einer zweiten Drosseleinrichtung 44, wo sie sich dahinter über eine dritte Verzweigungsleitung 22a in das erste Steuerventil 10.1 und eine vierte Verzweigungsleitung 22b in das zweite Steuerventil 10.2 verzweigt. Die Verzweigungsleitung 22a ist an einen nicht dargestellten Steuerzylinder der Zerstäuberdüsen 3.1 und die Verzweigungsleitung 22b ist an einen nicht dargestellten Steuerzylinder der Zerstäuberdüse 3.2 weitergeführt. Die vierte Verzweigungsleitung 22b ist nicht durchgehend dargestellt; ihre beiden Schnittstellen sind mit a gekennzeichnet. Bei den beiden Steuerventilen 10.1, 10.2 handelt es sich vorzugsweise um sog. 3/2-Wegeventile, deren Beschaltung und Funktion jedem Steuerungsfachmann geläufig ist und an dieser Stelle nicht näher erläutert wird. Von der zweiten Leitung 22 zweigt eine dritte Leitung 23 ab, die über ein sechstes Absperrventil 45 und eine dritte Drosseleinrichtung 46 geführt ist und die einen Flüssigkeitsspiegel im Vorratsbehälter 4 mit Druckluft AR eines vorgegebenen definierten Druckes beaufschlagt. Diese Druckbeaufschlagung ist für die Förderung des flüssigen zweiten Bestandteils F2' (z.B. flüssiges H2O2') zu den Zerstäuberdüsen 3.1, 3.2 verantwortlich.

Der jeweilige Anschluss zur Zufuhr des gasförmigen Bestandteils F1 (z.B. Luft AR oder Sterilluft AS) an der Zerstäuberdüse 3.1, 3.2 ist mit einer ersten Verzweigungsleitung 21a bzw. einer zweiten Verzweigungsleitung 21b verbunden, wobei letztere nicht durchgehend dargestellt ist; ihre beiden Schnittstellen sind mit b gekennzeichnet. Die beiden Verzweigungsleitungen 21a und 21b werden aus einer ersten Leitung 21 gespeist, die an der Verzweigungsstelle c an die Zuführleitung 20 für den gasförmigen ersten Bestandteil F1 (z.B. Luft Ar oder Sterilluft AS) angeschlossen ist. Ausgehend von dieser Verzweigungsstelle c befindet sich in der ersten Leitung 21, in Strömungsrichtung gesehen, ein fünftes Absperrventil 39, eine erste Drosseleinrichtung 40, ein Vorwärmer 9, eine erste Temperatur-Messeinrichtung 15 zur Messung einer Temperatur ϑ₁ = ϑ_{AR}, (ϑ_{AS}) und ein Regelventil 41 zur Regelung eines Druckes p₂ = p_{A} im Zerstäuber- und Speicherbehälter 1.1. Letzterem ist eine Druckregelungs-Einrichtung 14 zugeordnet, die einerseits über eine Messleitung 14a den Druck p₂ = p_{A} im Zerstäuber- und Speicherbehälter 1.1 misst und das daraus abgeleitete Steuersignal über eine Signalleitung 14b an das Regelventil 41 leitet.

Der Zerstäuber- und Speicherbehälter 1.1 **(****Figuren 2, 2a****;** **Figur 1****)** ist mit einer in diesen über eine Durchführung 1.6 hineingeführten Reinigungseinrichtung 19 versehen, die über eine Reinigungsmittelleitung 48 mit einem geeigneten Reinigungsmittel zur Durchflussreinigung des Behälters 1 und der aus dem Verdampferbehälter 1.2 ausmündenden und fortgeführten Leitungen 26, 28 versorgt wird. Der obere Boden des Zerstäuber- und Speicherbehälters 1.1 weist einen ersten Anschlussstutzen 1.7 zum Anschluss der Messleitung 14a, einen zweiten Anschlussstutzen 1.8 zum Anschluss einer zu einem ersten Sicherheitsventil 42 führenden Sicherheitsleitung 42a und ein erstes Schauglas 1.4 auf. Im Mantel des Zerstäuber- und Speicherbehälters 1.1 ist ein zweites Schauglas 1.5 angeordnet.

Die Funktions- und Arbeitsweise der erfindungsgemäßen Vorrichtung werden nachfolgend in einem von ihr eingenommenen quasi stationären Betriebszustand erläutert, wenn das zu dosierende sterilisierende Gasgemisch G hinsichtlich des erforderlichen festen Mengenverhältnisses F1/F2" bzw. F1/F2' einjustiert ist und sich die von der Vorrichtung mit sterilisierendem Gasgemisch G versorgte Verpackungsmaschine gleichfalls in einem quasi stationären Betriebszustand befindet. Letzterer bedeutet konkret, dass mit einer Taktfrequenz von beispielsweise 40 Takte/min eine Vielzahl von Verpackungsmittel, beispielsweise 16 kleine Flaschen oder Becher, parallel zur gleichzeitigen Spülung und Befüllung mit dem sterilisierenden Gasgemisch G über die Abgabeeinrichtung 30 (Dosierventil) an der Dosierstelle D bereitgestellt werden. Das Verhalten und die Betriebsweise der erfindungsgemäßen Vorrichtung bei länger andauernden Unterbrechungen und Störungen des Betriebs der Verpackungsmaschine soll hier nicht Gegenstand der Erörterung sein. Gleichwohl verfügt die in ihrem Aufbau vorstehend beschriebene erfindungsgemäße Vorrichtung über sämtliche Mittel, um solche Betriebszustände sachgerecht zu beherrschen.

Die anfängliche Justierung der Vorrichtung zum Erzeugen des Aerosols A mit dem erforderlichen festen Mengenverhältnis F1/F2' erfolgt dadurch, dass der am Ort der Verwendung des Gasgemischs G ausgebrachte Istwert des Mengenverhältnisses messtechnisch ermittelt wird. Dies kann auf einfache Weise dadurch erfolgen, dass das begaste Verpackungsmittel ohne Produkt verschlossen und sein Inhalt hinsichtlich des vorliegenden Mengenverhältnisses analysiert wird. Dieser Messwert wird dann zur Voreinstellung der dem festen Speichervolumen V zugeführten Volumenströme des gasförmigen ersten Bestandteils F1 und des flüssigen zweiten Bestandteils F2' verwendet.

Die justierte und im Dosierbetrieb befindliche Vorrichtung gemäß der Erfindung würde bei der vorstehend beispielhaft genannten hohen Taktfrequenz das sterilisierende Gasgemisch G quasi kontinuierlich an der Abgabeeinrichtung 30 bereitstellen. Bei Verpackungsmitteln größeren Inhalts, beispielsweise 1 oder 1,5 Liter PET Flaschen, wären die Taktfrequenz und die Zeiten der Nichtdosierung zwar deutlich niedriger, der auszubringende Volumenstrom jedoch nicht signifikant größer. Für jede dieser intermittierenden Abfuhren des sterilisierenden Gasgemischs G ist die erfindungsgemäße Vorrichtung geeignet und einstellbar.

Zentrale Bedeutung hat das Erzeugen des Aerosols A im Zerstäuber- und Speicherbehälter 1.1. Ein dort im hinreichenden festen Mengenverhältnis F1/F2' bereitgestelltes Aerosol A wird aufgrund der zwischen Zerstäuber- und Speicherbehälter 1.1 und Dosierstelle D, an der beispielsweise ein Druck p_{D} herrscht, wirksamen Druckdifferenz p₂ - p_{D} den Verdampferbehälter 1.2 auf dem Weg über dessen Heizeinrichtung 2 durchströmen und sich zum Gasgemisch G umwandeln. Vorzugsweise ist die Heizeinrichtung 2 dampfbeheizt, bevorzugt mit Wasserdampf ST unter Sättigungsbedingungen, so dass am Austritt eines bevorzugt zur Anwendung kommenden Rohrbündel-Wärmeaustauschers 2.1 die erforderliche Temperatur ϑ₃ = ϑ_{G} sehr genau gegeben ist. Da der Druck p₂ = p_{A} vorgegeben und konstant gehalten wird, ist das unter den gegebenen Dosierbedingungen je Takt abgegebene Gasgemisch G volumenmäßig eindeutig definiert, bestimmbar und reproduzierbar.

In Abhängigkeit von der intermittierenden Abfuhr des Gasgemischs G werden von der Abgabeeinrichtung 30, vorzugsweise über deren Rückmeldesignale in der Schließ- und Offenstellung, die Zerstäuberdüsen 3.1 und 3.2 gleichzeitig angesteuert. Hierzu dienen die Steuerventile 10.1 und 10.2. Da die Zulaufbedingungen für den an den Zerstäuberdüsen 3.1, 3.2 anstehenden gasförmigen ersten Bestandteil F1 (z.B. Luft AR oder Sterilluft AS) und für den flüssigen zweiten Bestandteil F2' (flüssiges H2O2') gleichfalls eindeutig definiert, bestimmbar und reproduzierbar sind, lässt sich das erforderliche feste Mengenverhältnis F1/F2' an den Zerstäuberdüsen 3.1, 3.2, die vorzugsweise als pneumatisch steuerbare Zweistoffdüsen ausgebildet sind, exakt einstellen. Als Stellgrößen für die Einstellung dieses Mengenverhältnisses dienen die veränderbare Größe und/oder die veränderbare Zeitdauer eines Öffnungshubs eines Schließgliedes der Zweistoffdüse. Der Volumenstrom des gasförmigen ersten Bestandteils F1 ist durch die Rohrleitungskennlinie und die Strömungsbedingungen zwischen der Zuführleitung 20 und der Austrittsstelle in den Zerstäuber- und Speicherbehälter 1.1 im Bereich der Zerstäuberdüsen 3.1, 3.2 eindeutig bestimmt.

Anzumerken wäre, dass der gasförmige erste Bestandteil F1 über den Vorwärmer 9 geführt und dort auf eine Temperatur ϑ₁ = ϑ_{AR} (vorzugsweise ca. 130 °C) erwärmt wird, die über der späteren Temperatur ϑ₃ = ϑ_{G} des sterilisierfähigen Gasgemischs G liegt und somit eine hinreichende Absorptionsfähigkeit der kontinuierlichen Phase F1 für die disperse Phase F2' sicherstellt und zu einer Verdampfung von Teilmengen oder auch bereits zur vollständigen Verdampfung der dispersen Phase F2', des flüssigen zweiten Bestandteils, bereits im Zerstäuber- und Speicherbehälter 1.1 führt. Im Verdampferbehälter 1.2, der sich dem Abführungsende des Speicherbehälters 1.1 anschließt, wird über die Heizeinrichtung 2 eine Oberflächentemperatur der Heizflächen von maximal 105 °C eingestellt, die eine schonende Aufheizung des Aerosols A bei Teilverdampfung im Speicherbehälter 1.1 oder des Gasgemischs G bei vollständiger Verdampfung im Speicherbehälter 1.1 bewirkt. Schließlich steht am Austritt des Verdampferbehälters 1.2 ein sterilisierfähiges, tröpfchenfreies Gasgemisch G, bestehend aus dem gasförmigen ersten Bestandteil F1, der Luft, und dem dampfförmigen zweiten Bestandteil F2", dem H2O2", zur Verfügung, das dann mit einer Temperatur von beispielsweise 80 °C in das zu sterilisierende Verpackungsmaterial eingebracht wird.

Der Volumenstrom des flüssigen zweiten Bestandteils F2' ist durch die Rohrleitungskennlinie und die Strömungsbedingungen zwischen dem Vorratsbehälter 4 und der Austrittsstelle in den Zerstäuber- und Speicherbehälter 1.1 im Bereich der Zerstäuberdüsen 3.1, 3.2 ebenfalls eindeutig bestimmt, da der im Vorratsbehälter 4 vorgelegte flüssige zweite Bestandteil F2' auf seiner Flüssigkeitsoberfläche mit Druckluft eines vorbestimmten definierten Druckes beaufschlagt ist.

Die dem Behälter 1 zugeordnete Druckregelungs-Einrichtung 14 hat Zugriff auf das Regelventil 41 in der ersten Leitung 21, die die Zerstäuberdüsen 3.1, 3.2 mit dem gasförmigen ersten Bestandteil F1 versorgt, und bewirkt dadurch, dass durch die Zufuhr der Stoffströme F1, F2' in den und die Abfuhr des Stoffstromes (Gasgemisch) G aus dem Behälter 1 ggf. initiierte Druckschwankungen dort zeitnah ausgeregelt werden.

Die vorbeschriebene erfindungsgemäß Vorrichtung ist mit ihren Mess- und Regeleinrichtungen befähigt, laufend Daten der Stoffströme zu ermitteln und/oder zu messen, mit denen eine Bilanzierung dieser Stoffströme und damit eine Kontrolle des besagten festen Mengenverhältnisses F1/F2' fortlaufend zeitnah erfolgen kann. Hierzu ist vorgesehen, dass über eine vorbestimmte erste Zeitdauer Δt der Verwendung des Gasgemischs G der Volumenstrom des gasförmigen ersten Bestandteils F1 ermittelt und der Volumenstrom des flüssigen zweiten Bestandteils F2' gemessen werden. Der Volumenstrom des Gasgemischs G ergibt sich aus der Rohrleitungskennlinie, den Strömungsbedingungen und der Taktfrequenz und der Anzahl der Takte an der Dosierstelle D. Aus den ermittelten und gemessenen Werten wird der Istwert des Mengenverhältnis F1/F2' berechnet. Bei einer Abweichung vom vorgegebenen festen Mengenverhältnis (Sollwert) (F1/F2') um ein vorgegebenes Toleranzintervall, die eine Störung der Dosierung bedeuten kann, wird ein Alarm- und/oder Abschaltsignal generiert.

Darüber hinaus bietet die erfindungsgemäße Vorrichtung die Möglichkeit, in bestimmten Zeitabständen eine zusätzliche Kontrolle des Dosierbetriebes vorzunehmen, indem der Verbrauch an flüssigem zweiten Bestandteil F2' herangezogen wird. Hierzu ist vorgesehen, dass für eine vorgegebene Änderung des Füllstandes in dem Vorratsbehälter 4 für den flüssigen zweiten Bestandteil F2' die hierfür notwendige zweite Zeitdauer Δt_{F} gemessen wird und letztere zur Kontrolle des gemessenen Volumenstromes des flüssigen zweiten Bestandteils F2' und/oder des vorgegebenen festen Mengenverhältnisses F1/F2' (Sollwert) herangezogen wird.

Aus dem oben Genannten wird verständlich, dass verschiedene Modifikationen und Varianten realisiert werden können, ohne vom Geist und dem neuen Konzept der vorliegenden Erfindung abzuweichen. Dies ist so zu verstehen, dass keine Beschränkung auf die speziellen Ausführungsformen beabsichtigt ist, welche hier illustriert worden sind. Die Offenbarung soll alle solchen Abwandlungen umfassen, die sich innerhalb des von den Ansprüchen beanspruchten Schutzbereichs befinden.

### BEZUGSZEICHENLISTE DER VERWENDETEN ABKÜRZUNGEN

- 1: Behälter
- 1.1: Zerstäuber- und Speicherbehälter
- 1.2: Verdampferbehälter
- 1.3: Austrittsstutzen
- 1.4: erstes Schauglas
- 1.5: zweites Schauglas
- 1.6: Durchführung
- 1.7: erster Anschlussstutzen
- 1.8: zweiter Anschlussstutzen

- 2: Heizeinrichtung

- 2.1: Rohrbündel-Wärmeaustauscher
- 2.1a: Innenrohre
- 2.1b: obere Rohrträgerplatte
- 2.1c: untere Rohrträgerplatte
- 2.1d: Zulaufstutzen
- 2.1e: Ablaufstutzen

- 2.2: geradliniges Rohr
- 2.3: gewendeltes Rohr

- 3: Zerstäuberdüse (gesteuert)
- 3.1: erste Zerstäuberdüse
- 3.2: zweite Zerstäuberdüse

- 4: Vorratsbehälter (H2O2)
- 5: Bereitstellungsbehälter (H2O2)
- 6: Fördereinrichtung
- 7: Rückschlagventil
- 8: Sieb
- 9: Vorwärmer

- 10: Steuerventil
- 10.1: erstes Steuerventil
- 10.2: zweites Steuerventil

- 11: Durchflussmesser
- 11.1: erster Durchflussmesser
- 11.2: zweiter Durchflussmesser

- 12: Füllstandsanzeige-Einrichtung
- 13: Füllstandsregelungs-Einrichtung

- 14: Druckregelungs-Einrichtung
- 14a: Messleitung
- 14b: Signalleitung

- 15: erste Temperatur-Messeinrichtung (Temperatur des gasförmigen ersten Bestandteils F1 (der Luft) ϑ₁ = ϑ_{AR}, ϑ_{AS})
- 16: zweite Temperatur-Messeinrichtung (Temperatur des Aerosols ϑ₂ = ϑ_{A})
- 17: dritte Temperatur-Messeinrichtung (Temperatur des Gasgemischs ϑ₃ = ϑ_{G})
- 18: Druck-Messeinrichtung (Dampfdruck p₃ = p_{ST})
- 19: Reinigungseinrichtung
- 20: Zuführleitung (Luft AR; Sterilluft AS)

- 21: erste Leitung
- 21a: erste Verzweigungsleitung
- 21b: zweite Verzweigungsleitung

- 22: zweite Leitung
- 22a: dritte Verzweigungsleitung
- 22b: vierte Verzweigungsleitung

- 23: dritte Leitung
- 24: Füllleitung

- 25: vierte Leitung
- 25a: fünfte Verzweigungsleitung
- 25b: sechste Verzweigungsleitung

- 26: Dosierleitung
- 27: Umgehungsleitung
- 28: erste Rückführleitung

- 29a: Zulaufleitung
- 29b: Ablaufleitung

- 30: Abgabeeinrichtung (Dosierventil)
- 31: erstes Absperrventil
- 32: zweites Absperrventil
- 33: erster Kondensatabscheider
- 34: zweiter Kondensatabscheider
- 35: drittes Absperrventil
- 36: viertes Absperrventil
- 37: erster Ablauf
- 38: zweiter Ablauf
- 39: fünftes Absperrventil
- 40: erste Drosseleinrichtung
- 41: Regelventil

- 42: erstes Sicherheitsventil
- 42a: Sicherheitsleitung

- 43: zweites Sicherheitsventil
- 44: zweite Drosseleinrichtung
- 45: sechstes Absperrventil

- 46: dritte Drosseleinrichtung
- 47: vierte Drosseleinrichtung
- 48: Reinigungsmittelleitung
- 49: zweite Rückführleitung

- A: Aerosol (disperses System aus kontinuierlicher Phase F1 (AR; AS) und disperser Phase F2' (flüssiges H2O2'))

- AR: Luft (kontinuierliche Phase)
- AS: Sterilluft (kontinuierliche Phase)

- B: Abluft (bestehend aus Luft AR oder Sterilluft AS und/oder Gasgemisch G)

- D: Dosierstelle

- F: Fluid (einphasig, bestehend aus F1 und F2")

- F1: gasförmiger erster Bestandteil (kontinuierliche Phase; Luft AR; Sterilluft AS)

- F2: zweiter Bestandteil allgemein (z.B. H2O2)
- F2': flüssiger zweiter Bestandteil (disperse Phase; flüssiges H2O2')
- F2": dampfförmiger zweiter Bestandteil (dampfförmiges H2O2)

- G: Gasgemisch (Einphasensystem; Luft AR oder Sterilluft AS und dampfförmiges H2O2")

- H2O2: Wasserstoffperoxid (H₂O₂; allgemein)
- H2O2': flüssiges Wasserstoffperoxid (disperse Phase; flüssige Phase)
- H2O2": dampfförmiges Wasserstoffperoxid (dampfförmige Phase)

- L₁: erster Füllstand
- Lₒ₁: maximaler erster Füllstand (oberer Grenzwert)

- Lᵤ₁: minimaler erster Füllstand (unter Grenzwert)

- L₂: zweiter Füllstand
- Lₒ₂: maximaler zweiter Füllstand (oberer Grenzwert)
- Lᵤ₂: minimaler zweiter Füllstand (unterer Grenzwert)

- Q: gesamter Volumenstrom (flüssiges (Wasserstoffperoxid) H2O2')
- Q₁: erster Teilvolumenstrom (flüssiges H2O2')
- Q₂: zweiter Teilvolumenstrom (flüssiges H2O2')

- ST: Heißdampf (Wasserdampf)
- V: festes Speichervolumen des Behälters 1

- a: Schnittstelle der vierten Verzweigungsleitung 22b
- b: Schnittstelle der zweiten Verzweigungsleitung 21b
- c: Verzweigungsstelle

- p₂ = p_{A}: Druck im Zerstäuber- und Speicherbehälter
- p₃ = p_{ST}: Druck des Heißdampfes
- p_{D}: Druck an der Dosierstelle D

- ϑ₁ = ϑ_{AR}: erste Temperatur (Temperatur der vorgewärmten Luft AR)
- ϑ₂ = ϑ_{A}: zweite Temperatur (Temperatur des Aerosols A)
- ϑ₃ = ϑ_{G}: dritte Temperatur (Temperatur des Gasgemischs (H2O2"+AR))

- Δt: erste Zeitdauer (für Bilanzierung)
- Δt_{F}: zweite Zeitdauer (für eine vorgegebene Füllstandsänderung im Vorratsbehälter 4)

## Patentansprüche

1. Vorrichtung zum Erzeugen eines sterilisierenden Gasgemischs, in der das Gasgemisch (G) aus einem gasförmigen ersten Bestandteil (F1) als kontinuierlicher Phase, insbesondere Luft (AR) wie Sterilluft (AS), und einem flüssigen zweiten Bestandteil (F2') als disperser Phase, insbesondere einem Sterilisationsmittel wie Wasserstoffperoxid (H2O2'), in einem vorgegebenen Mengenverhältnis (F1/F2') generiert wird, mit einem Zerstäuber- und Speicherbehälter (1.1) mit einem festen Speichervolumen (V), in dem der flüssige zweite Bestandteil (F2') zerstäubt und mit dem gasförmigen Bestandteil (F1) homogen vermischt wird, wobei das so erzeugte disperse System, das Aerosol (A; F1, F2'; AR, H2O2'; AS, H2O2'), in dem festen Speichervolumen (V) unter konstantem Druck steht und das gewonnene Fluid (A; F) einer Abgabeeinrichtung (30) zugeführt und von dieser bedarfsweise gesteuert abgeführt wird, und wobei der Druckverlust im Speicherbehälter (1.1) beim Öffnen der Abgabeeinrichtung (30) durch Nachlieferung des gasförmigen ersten Bestandteils (F1) unter gleichzeitiger Zuführung und Beimischung des flüssigen zweiten Bestandteils (F2') in dem vorgegebenen Mengenverhältnis (F1/F2') kompensiert wird, wobei ein Behälter (1) in seinem oberen Teil aus dem Zerstäuber- und Speicherbehälter (1.1) besteht, der unterhalb an seinem Abführungsende in einen Verdampferbehälter (1.2) mit einer Heizeinrichtung (2) übergeht, dass der Zerstäuber- und Speicherbehälter (1.1) wenigstens eine gesteuerte Zerstäuberdüse (3; 3.1, 3.2) aufweist, die einen Anschluss zur Zufuhr des gasförmigen ersten Bestandteils (F1) und einen Anschluss zur Zufuhr des flüssigen zweiten Bestandteils (F2') besitzt, und dass dem Behälter (1) eine Druckregelungs-Einrichtung (14) zugeordnet ist, die in Abhängigkeit von den Anforderungen der Abgabeeinrichtung (30) die wenigstens eine Zerstäuberdüse (3; 3.1, 3.2) ansteuert und durch Zufuhr von gasförmigem ersten Bestandteil (F1) und flüssigem zweiten Bestandteil (F2') im vorgegebenen Mengenverhältnis (F1/F2') den vorgegebenen konstanten Druck (p₂ = p_{A}) im Behälter (1) aufrecht erhält,
**dadurch gekennzeichnet,**
**dass** die Abgabeeinrichtung (30) ein Dosierventil umfasst, welches über eine Dosierleitung (26) mit dem Verdampferbehälter (1.2) verbunden ist.

2. Vorrichtung nach Anspruch 1,
**dadurch gekennzeichnet,**
**dass** der Verdampferbehälter (1.2) in Form eines gegenüber dem Zerstäuber- und Speicherbehälter (1.1) verjüngten rohrförmigen Behälterteils ausgebildet ist.

3. Vorrichtung nach Anspruch 1 oder 2,
**dadurch gekennzeichnet,**
**dass** die Heizeinrichtung (2) wenigsten ein von außen beheizbares Rohr (2.1; 2.2; 2.3) aufweist, in dem das zu beheizende Aerosol (A) oder Fluid (F) strömt, wobei der Außenmantel der Heizeinrichtung (2), der ein Heizmedium innenseits aufnimmt, vom Mantel des Verdampferbehälters (1.2) gebildet ist.

4. Vorrichtung nach Anspruch 1 oder 2
**dadurch gekennzeichnet,**
**dass** die Heizeinrichtung (2) als Rohrbündel-Wärmeaustauscher (2.1) ausgebildet ist, in dem das zu beheizende Aerosol (A) oder Fluid (F) auf der Innenseite von wenigstens zwei parallel geschalteten Innenrohren (2.1a) strömt, wobei der Außenmantel des Rohrbündel-Wärmeaustauschers (2.1), der ein Heizmedium innenseits aufnimmt, vom Mantel des Verdampferbehälters (1.2) gebildet ist.

5. Vorrichtung nach Anspruch 4,
**dadurch gekennzeichnet,**
**dass** sich der Rohrbündel-Wärmeaustauscher (2.1) über die gesamte Länge des Verdampferbehälters (1.2) erstreckt.

6. Vorrichtung nach Anspruch 3,
**dadurch gekennzeichnet,**
**dass** das Rohr als geradliniges Rohr (2.2) ausgeführt ist.

7. Vorrichtung nach Anspruch 3,
**dadurch gekennzeichnet,**
**dass** das Rohr als gewendeltes Rohr (2.3) ausgeführt ist.

8. Vorrichtung nach einem der Ansprüche 1 bis 7,
**dadurch gekennzeichnet,**
**dass** die Heizeinrichtung (2) dampfbeheizt ist, vorzugsweise mit Wasserdampf ST unter Sättigungsbedingungen.

9. Vorrichtung nach einem der Ansprüche 1 bis 8,
**dadurch gekennzeichnet,**
**dass** im Mantel des Zerstäuber- und Speicherbehälters (1.1) zwei Zerstäuberdüsen angeordnet sind.

10. Vorrichtung nach Anspruch 9,
**dadurch gekennzeichnet,**
**dass** die Zerstäuberdüsen (3.1, 3.2) diametral zueinander angeordnet sind.

11. Vorrichtung nach Anspruch 10,
**dadurch gekennzeichnet,**
**dass** die Zerstäuberdüsen (3.1, 3.2) in der Höhe axial versetzt zueinander angeordnet sind.

12. Vorrichtung nach einem der Ansprüche 1 bis 11,
**dadurch gekennzeichnet,**
**dass** die Zerstäuberdüse(n) (3; 3.1, 3.2) als Zweistoffdüse ausgebildet ist(sind).

13. Vorrichtung nach Anspruch 12,
**dadurch gekennzeichnet,**
**dass** das vorgegebene feste Mengenverhältnis (F1/F2') der durch die Zerstäuberdüse(n) (3; 3.1, 3.2) auszubringenden beiden Bestandteile (F1, F2') durch eine veränderbare Größe und/oder eine veränderbare Zeitdauer eines Öffnungshubes eines Schließgliedes der Zerstäuberdüse(n) (3; 3.1, 3.2) einstellbar ist.

14. Vorrichtung nach einem der Ansprüche 1 bis 13,
**dadurch gekennzeichnet,**
**dass** die Zerstäuberdüse(n) (3; 3.1, 3.2) pneumatisch angesteuert wird(werden).

15. Vorrichtung nach einem der Ansprüche 1 bis 14,
**dadurch gekennzeichnet,**
**dass** in jeder Leitung (25; 25, 25a, 25b), die als Zuleitung zu der(den) Zerstäuberdüse(n) (3; 3.1, 3.2) für den flüssigen Bestandteil (F2'; H2O2') fungiert, ein Durchflussmesser (11; 11, 11.1, 11.2) angeordnet ist.

16. Vorrichtung nach einem der Ansprüche 1 bis 15,
**dadurch gekennzeichnet,**
**dass** die Zerstäuberdüse(n) (3; 3.1, 3.2) über die Leitung(en) (25; 25, 25a, 25b) mit einem in seinem Füllstand geregelten Vorratsbehälter (4) für den flüssigen Bestandteil (F2'; H2O2') verbunden ist(sind).

17. Vorrichtung nach Anspruch 16,
**dadurch gekennzeichnet,**
**dass** eine dem Vorratsbehälter (4) zugeordnete Füllstandsregelungs-Einrichtung (13) für eine vorgegebene Füllstandsänderung Steuersignale generiert.

18. Vorrichtung nach einem der Ansprüche 1 bis 17,
**dadurch gekennzeichnet,**
**dass** in einer für die Zufuhr des gasförmigen ersten Bestandteils (F1) in den Speicherbehälter (1.1) vorgesehenen ersten Leitung (21) ein Vorwärmer (9) angeordnet ist.

19. Verfahren zum Erzeugen eines sterilisierenden Gasgemischs unter Verwendung einer Vorrichtung gemäß Anspruch 1, mit dem das Gasgemisch (G) aus einem gasförmigen ersten Bestandteil (F1) als kontinuierlicher Phase, insbesondere Luft (AR) wie Sterilluft (AS), und einem flüssigen zweiten Bestandteil (F2') als disperser Phase, insbesondere einem Sterilisationsmittel wie Wasserstoffperoxid (H2O2'), in einem vorgegebenen Mengenverhältnis (F1/F2') generiert wird, wobei der flüssige zweite Bestandteil (F2') in einem festen Speichervolumen unter konstantem Druck zerstäubt und mit dem gasförmigen ersten Bestandteil (F1) homogen vermischt und das so erzeugte disperse System, das Aerosol (A; F1, F2'; AR, H2O2'; AS, H2O2'), zum Zwecke der Sterilisation bzw. Desinfektion bedarfsorientiert gesteuert abgeführt und verwendet wird, und wobei der Druckverlust in dem festen Speichervolumen bei der Abfuhr des gewonnenen Fluids(A; F) durch Nachlieferung des gasförmigen ersten Bestandteils (F1) unter gleichzeitiger Zuführung und Beimischung des flüssigen zweiten Bestandteils (F2') in dem vorgegebenen Mengenverhältnis (F1/F2') kompensiert wird, wobei das Aerosol (A) in dem festen Speichervolumen (V) bei dem dort vorgegebenen und konstant gehaltenen Druck (p₂ = p_{A}) dergestalt bereitgestellt wird, dass die bedarfsorientierte gesteuerte Abfuhr des Aerosols (A) aus dem Speichervolumen (V) durch eine in Abhängigkeit von der intermittierenden Abfuhr gesteuerte Zufuhr des gasförmigen ersten Bestandteils (F1) und des flüssigen zweiten Bestandteils (F2'), die in dem vorgegebenen festen Mengenverhältnis (F1/F2') zueinander stehen, in das Speichervolumen (V) zeitnah ausgeglichen wird, und dass das Aerosol (A) zum Verdampfen des flüssigen zweiten Bestandteils (F2') oder das gewonnene gasförmige Fluid (F) anschließend auf seinem weiteren Strömungsweg indirekt beheizt und das schließlich gewonnene Gasgemisch (G), gebildet aus dem gasförmigen ersten Bestandteil (F1) und dem dampfförmigen zweiten Bestandteil (F2"; H2O2"), seiner Zweckbestimmung zugeführt wird.

20. Verfahren nach Anspruch 19,
**dadurch gekennzeichnet,**
**dass** das Aerosol (A) mittels Heißdampf (ST), insbesondere Wasserdampf im Sättigungszustand, beheizt wird.

21. Verfahren nach Anspruch 19 oder 20,
**dadurch gekennzeichnet,**
**dass** die Beheizung mit einer Oberflächentemperatur von maximal 105 °C erfolgt.

22. Verfahren nach einem der Ansprüche 19 bis 21,
**dadurch gekennzeichnet,**
**dass** der gasförmige erste Bestandteil (F1) vor der Beimengung des flüssigen zweiten Bestandteils (F2') vorgewärmt wird.

23. Verfahren nach Anspruch 22,
**dadurch gekennzeichnet,**
**dass** die Vorwärmung auf eine Temperatur ϑ₁ = ϑ_{AR} (ϑ_{AS}) erfolgt, die über der späteren Temperatur ϑ₃ = ϑ_{G} des Gasgemischs (G) liegt, bevorzugt auf ϑ₁ = 130 °C.

24. Verfahren nach einem der Ansprüche 19 bis 23,
**dadurch gekennzeichnet,**
**dass** der am Ort der Verwendung des Gasgemischs (G) ausgebrachte Istwert des Mengenverhältnis (F1/F2') messtechnisch ermittelt und dieser Messwert zur Voreinstellung der dem Speichervolumen (V) zugeführten Volumenströme des gasförmigen ersten Bestandteils (F1) und des flüssigen zweiten Bestandteils (F2') verwendet wird.

25. Verfahren nach einem der Ansprüche 19 bis 24,
**dadurch gekennzeichnet,**
**dass** über eine vorbestimmte erste Zeitdauer (Δt) der Verwendung des Gasgemischs (G) der Volumenstrom des gasförmigen ersten Bestandteils (F1) ermittelt und der Volumenstrom des flüssigen zweiten Bestandteils (F2') gemessen werden, dass aus diesen Werten der Istwert des Mengenverhältnis (F1/F2') berechnet wird, und dass bei einer Abweichung vom vorgegebenen festen Mengenverhältnis (Sollwert) (F1/F2') um ein vorgegebenes Toleranzintervall ein Alarm- und/oder Abschaltsignal generiert wird.

26. Verfahren nach einem der Ansprüche 19 bis 25,
**dadurch gekennzeichnet,**
**dass** für eine vorgegebene Änderung des Füllstandes in einem Vorratsbehälter (4) für den flüssigen zweiten Bestandteil (F2') eine hierfür notwendige zweite Zeitdauer (Δt_{F}) gemessen wird und letztere zur Kontrolle des gemessenen Volumenstromes des flüssigen zweiten Bestandteils (F2') und/oder des vorgegebenen festen Mengenverhältnisses (F1/F2') (Sollwert) herangezogen wird.

27. Verwendung einer Vorrichtung nach einem oder mehreren der Ansprüche 1 bis 18 bei einer Verpackungsmaschine zum Sterilisieren des Verpackungsmittels oder der durch Behälterwände begrenzten Verpackungszone.

## Claims

1. An apparatus for the production of a sterilizing gas mixture, in which the gas mixture (G) is generated from a gaseous first component (F1) as continuous phase, in particular air (AR) like sterilized air (AS), and a liquid second component (F2') as disperse phase, in particular a sterilization means like hydrogen peroxide (H2O2'), in a predetermined proportion (F1/F2'), with an atomizer and storage container (1.1) with a fixed storage volume (V), in which the liquid second component (F2') is atomized and mixed homogenously with the gaseous component (F1), wherein the disperse system created in this manner, the aerosol (A; F1, F2'; AR, H2O2'; AS, H2O2'), in the fixed storage volume (V) is under constant pressure and the so obtained fluid (A; F) is fed to a dispensing device (30) and is discharged from it as needed in a controlled manner, and wherein the pressure decrease in the storage container (1.1) is compensated during the opening of the delivery device (30) through subsequent delivery of the gaseous first component (F1) under simultaneous supply and admixture ofthe liquid second component (F2') in the predetermined proportion (F1/F2'), wherein a container (1) in its upper part consists ofthe atomizer and storage container (1.1), which on the bottom on its discharge end merges into a vaporizer container (1.2) with a heating device (2), that the atomizer and storage device (1.1) has at least one controlled atomizer nozzle (3; 3.1, 3.2), which has a connection to the supply of the gaseous first component (F1) and a connection to the supply of the liquid second component (F2'), and that a pressure regulation device (14) is assigned to the container (1), which depending on the requirements ofthe dispensing device (30) controls the at least one atomizer nozzle (3; 3.1, 3.2) and maintains the predetermined constant pressure (p₂ = p_{A}) in the container (1) through the supply of gascous first component (F1) and liquid second component (F2') in the predetermined proportion (F1/F2'),
**characterized in that**
the delivery device (30) comprises a dosing valve, which is connected with the vaporizer container (1.2) via a dosing line (26).

2. The apparatus according to claim 1,
**characterized in that**
the vaporizer container (1.2) is designed in the shape of a tubular container part tapered relative to the atomizer and storage container (1.1).

3. The apparatus according to claim 1 or 2,
**characterized in that**
the heating device (2) has at least one tube (2.1; 2.2; 2.3) heatable from the outside, in which the aerosol (A) or fluid (F) to be heated flows, wherein the outer casing of the heating device (2), which receives a heating medium on the inside, is formed by the casing of the vaporizer container (1.2).

4. The apparatus according to claim 1 or 2,
**characterized in that**
the heating device (2) is designed as a tube bundle heat exchanger (2.1), in which the aerosol (A) or fluid (F) to be heated flows on the inside of at least two parallel switched inner tubes (2.1a), wherein the outer casing of the tube bundle heat exchanger (2.1), which receives a heating medium on the inside, is formed by the casing ofthe vaporizer container (1.2).

5. The apparatus according to claim 4,
**characterized in that**
the tube bundle heat exchanger (2.1) extends over the entire length of the vaporizer container (1.2).

6. The apparatus according to claim 3,
**characterized in that**
the tube is designed as a straight tube (2.2).

7. The apparatus according to claim 3,
**characterized in that**
the tube is designed as a coiled tube (2.3).

8. The apparatus according to one of claims 1 through 7,
**characterized in that**
the heating device (2) is steam heated, preferably with steam ST under saturated conditions.

9. The apparatus according to one of claims I through 8,
**characterized in that**
two atomizer nozzles are arranged in the casing ofthe atomizer and storage container (1.1).

10. The apparatus according to claim 9,
**characterized in that**
the atomizer nozzles (3.1, 3.2) are arranged diametrically opposed to each other.

11. The apparatus according to claim 10,
**characterized in that**
the atomizer nozzles (3.1, 3.2) are arranged axially offset from each other in terms of height.

12. The apparatus according to one of claims 1 through 11,
**characterized in that**
the atomizer nozzle(s) (3; 3.1, 3.2) is (are) designed as a two-component nozzle.

13. The apparatus according to claim 12,
**characterized in that**
the predetermined fixed proportion (F1/F2') of the two components (F1, F2') to be delivered through the atomizer nozzle(s) (3; 3.1, 3.2) is adjustable through a changeable variable and/or a changeable duration of an opening stroke of a closing member of the atomizer nozzle(s) (3; 3.1, 3.2).

14. The apparatus according to one of claims I through 13,
**characterized in that**
the atomizer nozzle(s) (3; 3.1, 3.2) is (are) pneumatically driven.

15. The apparatus according to one of claims I through 14,
**characterized in that**
a flow meter (11; 11, 11.1, 11.2) is arranged in each line (25; 25, 25a, 25b), which functions as supply line to the atomizer nozzle(s) (3; 3.1, 3.2) for the liquid component (F2'; H2O2').

16. The apparatus according to one of claims I through 15,
**characterized in that**
the atomizer nozzle(s) (3; 3.1, 3.2) is (are) connected with a fill-level-regulated storage container (4) for the liquid component (F2'; H2O2') via the line(s) (25; 25, 25a, 25b).

17. The apparatus according to claim 16,
**characterized in that**
a fill-level regulation device (13) assigned to the storage container (4) generates control signals for a predetermined fill level change.

18. The apparatus according to one of claims 1 through 17,
**characterized in that**
a preheater (9) is arranged in a first line (21) provided for the supply of the gaseous first component (F1) to the storage container (1.1).

19. Method for the production of a sterilizing gas mixture using an apparatus according to claim 1, with which the gas mixture (G) is generated from a gaseous first component (F1) as continuous phase, in particular air (AR) like sterilized air (AS), and a liquid second component (F2') as disperse phase, in particular a sterilization means like hydrogen peroxide (H2O2'), in a predetermined proportion (F1/F2'), wherein the liquid second component (F2') is atomized in a fixed storage volume under constant pressure and mixed homogenously with the gaseous first component (F1) and the disperse system created in this manner, the aerosol (A; F1, F2'; AR, H2O2'; AS, H2O2') is discharged and used in a controlled manner depending on requirements for the purpose of sterilization or respectively disinfection, and wherein the pressure decrease in the fixed storage volume is compensated during discharge of the obtained fluid (A; F) through subsequent delivery of the gaseous first component (F1) under simultaneous supply and admixture of the liquid second component (F2') in the predetermined proportion (F1/F2'), wherein the aerosol (A) in the fixed storage volume (V) is provided in the case of the predetermined and constant pressure (p₂ = p_{A}) such that the requirements-based controlled discharge ofthe aerosol (A) from the storage volume (V) is compensated through a supply of the gaseous first component (F1) and of the liquid second component (F2') controlled depending on the intermittent discharge, which are related in the predetermined fixed proportion (F1/F2'), in that storage volume (V) is balanced promptly, and that the aerosol (A) for vaporization ofthe liquid second component (F2') or the obtained gaseous fluid (F) is indirectly heated subsequently on its further flow path and the finally obtained gas mixture (G), formed from the gaseous first component (F1) and the vaporous second component (F2"; H2O2"), is supplied for its specific function.

20. The method according to claim 19,
**characterized in that**
the aerosol (A) is heated by means of heated steam (ST), in particular steam in the saturation state.

21. The method according to claim 19 or 20,
**characterized in that**
the heating takes place with a surface temperature of maximum 105° C.

22. The method according to one of claims 19 through 21,
**characterized in that**
the gaseous first component (F1) is preheated before the admixture of the liquid second component (F2').

23. The method according to claim 22,
**characterized in that**
the preheating takes place to a temperature ϑ₁ = ϑ_{AR} (ϑ_{AS}), which lies above the later temperature ϑ₃ = ϑ_{G} of the gas mixture (G), preferably at ϑ₁ = 130 °C.

24. The method according to one of claims 19 through 23,
**characterized in that**
the actual value of the proportion (F1/F2') yielded at the location of the use of the gas mixture (G) is determined metrologically and this measured value is used for the presetting of the volume flows of the gaseous first component (F1) and of the liquid second component (F2') supplied to the storage volume (V).

25. The method according to one of claims 19 through 24,
**characterized in that**
the volume flow of the gaseous first component (F1) is determined and the volume flow of the liquid second component (F2') is measured over a predetermined first duration (At) of the use of the gas mixture (F), that the actual value of the proportion (F1/F2') is calculated from these values, and that an alarm and/or shutoff signal is generated in the case of a deviation from the predetermined fixed proportion (setpoint value) (F1/F2') by a predetermined tolerance interval.

26. The method according to one of claims 19 through 25,
**characterized in that**
a second duration (Δt_{F}) necessary for a predetermined change in the fill level in a storage container (4) for the liquid second component (F2') is measured and the latter is used to check the measured volume flow of the liquid second component (F2') and/or the predetermined fixed proportion (F1/F2') (setpoint value).

27. Use of an apparatus according to one or more of claims 1 through 18 in a packaging machine for sterilizing the packaging means or the packaging zone bordered by container walls.

## Revendications

1. Dispositif de production d'un mélange gazeux stérilisant, dans lequel le mélange gazeux (G) composé d'un premier constituant (F1) gazeux en tant que phase continue, en particulier de l'air (AR) tel que de l'air stérile (AS), et d'un deuxième constituant (F2') liquide en tant que phase dispersée, en particulier un agent stérilisant tel que du peroxyde d'hydrogène (H2O2'), est produit dans un rapport quantitatif (F1/F2') prédéfini, avec un contenant de pulvérisation et d'accumulation (1.1) avec un volume d'accumulation (V) fixe, dans lequel le deuxième constituant (F2') liquide est pulvérisé et est mélangé de façon homogène avec le premier constituant (F1) gazeux, le système dispersé ainsi produit, l'aérosol (A ; F1, F2' ; AR, H2O2' ; AS, H2O2'), étant sous une pression constante dans le volume d'accumulation (V) fixe, et le fluide obtenu (A ; F) étant conduit à un dispositif de distribution (30) et en cas de besoin évacué de façon commandée par celui-ci, et la perte de pression dans le contenant d'accumulation (1.1) lors de l'ouverture du dispositif de distribution (30) étant compensée par la fourniture complémentaire du premier constituant (F1) gazeux accompagnée simultanément de l'amenée et du mixage du deuxième constituant (F2') liquide dans le rapport quantitatif (F1/F2') prédéfini, un contenant (1) étant dans sa partie supérieure composé du contenant de pulvérisation et d'accumulation (1.1) qui, au-dessous, au niveau de son extrémité d'évacuation, se transforme en un contenant d'évaporation (1.2) avec un dispositif de chauffage (2), le contenant de pulvérisation et d'accumulation (1.1) présentant au moins une buse de pulvérisation (3 ; 3.1, 3.2) commandée qui possède un raccord pour l'amenée du premier constituant (F1) gazeux et un raccord pour l'amenée du deuxième constituant (F2') liquide, un dispositif de régulation de pression (14) étant affecté au contenant (1) et pilotant la buse de pulvérisation (3 ; 3.1, 3.2) au moins au nombre de un en fonction des spécifications du dispositif de distribution (30) et maintenant, par l'amenée de premier constituant (F1) gazeux et de deuxième constituant (F2') liquide dans le rapport quantitatif (F1/F2') prédéfini dans le contenant (1), la pression (p₂ = p_{A}) constante prédéfinie,
**caractérisé en ce que**
le dispositif de distribution (30) comprend une vanne de dosage qui est raccordée au contenant d'évaporation (1.2) par le biais d'une conduite de dosage (26).

2. Dispositif selon la revendication 1,
**caractérisé en ce que**
le contenant d'évaporation (1.2) est réalisé sous la forme d'une partie de contenant tubulaire rétrécie par rapport au contenant de pulvérisation et d'accumulation (1.1).

3. Dispositif selon la revendication 1 ou 2,
**caractérisé en ce que**
le dispositif de chauffage (2) présente au moins un tube (2.1 ; 2.2 ; 2.3) pouvant être chauffé de l'extérieur, dans lequel s'écoule l'aérosol (A) ou le fluide (F) à chauffer, l'enveloppe extérieure du dispositif de chauffage (2) qui reçoit intérieurement un agent de chauffage étant formée de l'enveloppe du contenant d'évaporation (1.2).

4. Dispositif selon la revendication 1 ou 2,
**caractérisé en ce que**
le dispositif de chauffage (2) est réalisé en tant qu'échangeur de chaleur à faisceau tubulaire (2.1) dans lequel s'écoule l'aérosol (A) ou le fluide (F) à chauffer sur le côté intérieur d'au moins deux tubes intérieurs (2.1a) montés en parallèle, l'enveloppe extérieure de l'échangeur de chaleur à faisceau tubulaire (2.1) qui reçoit intérieurement un agent de chauffage étant formée de l'enveloppe du contenant d'évaporation (1.2).

5. Dispositif selon la revendication 4,
**caractérisé en ce que**
l'échangeur de chaleur à faisceau tubulaire (2.1) s'étend sur toute la longueur du contenant d'évaporation (1.2).

6. Dispositif selon la revendication 3,
**caractérisé en ce que**
le tube est exécuté en tant que tube (2.2) rectiligne.

7. Dispositif selon la revendication 3,
**caractérisé en ce que**
le tube est exécuté en tant que tube en hélice (2.3).

8. Dispositif selon une des revendications 1 à 7,
**caractérisé en ce que**
le dispositif de chauffage (2) est chauffé à la vapeur, de préférence à la vapeur d'eau ST dans des conditions de saturation.

9. Dispositif selon une des revendications 1 à 8,
**caractérisé en ce que**
deux buses de pulvérisation sont disposées dans l'enveloppe du contenant de pulvérisation et d'accumulation (1.1).

10. Dispositif selon la revendication 9,
**caractérisé en ce que**
les buses de pulvérisation (3.1, 3.2) sont disposées de façon diamétralement opposée.

11. Dispositif selon la revendication 10,
**caractérisé en ce que**
les buses de pulvérisation (3.1, 3.2) sont disposées de façon axialement décalée en hauteur l'une par rapport à l'autre.

12. Dispositif selon une des revendications 1 à 11,
**caractérisé en ce que**
la / les buse(s) de pulvérisation (3 ; 3.1, 3.2) est/sont réalisée(s) en tant que buse à deux composants.

13. Dispositif selon la revendication 12,
**caractérisé en ce que**
le rapport quantitatif (F1/F2') fixe prédéfini des deux constituants (F1, F2') devant être distribués par la/les buse(s) de pulvérisation (3 ; 3.1, 3.2) peut être réglé par une grandeur variable et/ou une durée variable d'une course d'ouverture d'un organe de fermeture de la / des buse(s) de pulvérisation (3 ; 3.1, 3.2).

14. Dispositif selon une des revendications 1 à 13,
**caractérisé en ce que**
la/les buse(s) de pulvérisation (3 ; 3.1, 3.2) est/sont pilotée(s) de façon pneumatique.

15. Dispositif selon une des revendications 1 à 14,
**caractérisé en ce**
**qu'**il est disposé un débitmètre (11 ; 11, 11.1, 11.2) dans chaque conduite (25 ; 25, 25a, 25b) qui fonctionne en tant que conduite d'amenée vers la/les buse(s) de pulvérisation (3 ; 3.1, 3.2) pour le constituant (F2' ; H2O2') liquide.

16. Dispositif selon une des revendications 1 à 15,
**caractérisé en ce que**
la/les buse(s) de pulvérisation (3 ; 3.1, 3.2) est/sont, par le biais de la/des conduites (25 ; 25, 25a, 25b), raccordée(s) à un réservoir de stockage (4) pour le deuxième constituant (F2' ; H2O2') liquide dont le niveau est régulé.

17. Dispositif selon la revendication 16,
**caractérisé en ce**
**qu'**un dispositif de régulation de niveau (13) affecté au réservoir de stockage (4) génère des signaux de commande pour une variation de niveau prédéfinie.

18. Dispositif selon une des revendications 1 à 17,
**caractérisé en ce**
**qu'**un réchauffeur (9) est disposé dans une première conduite (21) prévue pour l'amenée du premier constituant (F1) gazeux dans le contenant d'accumulation (1.1).

19. Procédé de production d'un mélange gazeux stérilisant avec utilisation d'un dispositif selon la revendication 1, avec lequel le mélange gazeux (G) composé d'un premier constituant (F1) gazeux en tant que phase continue, en particulier de l'air (AR) tel que de l'air stérile (AS), et d'un deuxième constituant (F2') liquide en tant que phase dispersée, en particulier un agent stérilisant tel que du peroxyde d'hydrogène (H2O2'), est produit dans un rapport quantitatif (F1/F2') prédéfini, le deuxième constituant (F2') liquide étant pulvérisé dans un volume d'accumulation fixe à une pression constante et mélangé de façon homogène avec le premier constituant (F1) gazeux, et le système dispersé ainsi produit, l'aérosol (A ; F1, F2' ; AR, H2O2' ; AS, H2O2'), étant évacué de façon commandée et utilisé en fonction des besoins à des fins de stérilisation ou de désinfection, et la perte de pression dans le volume d'accumulation fixe (1.1) lors de l'évacuation du fluide (A ; F) obtenu étant compensée simultanément par la fourniture complémentaire du premier constituant (F1) gazeux accompagnée simultanément de l'amenée et du mixage du deuxième constituant (F2') liquide dans le rapport quantitatif (F1/F2') prédéfini, l'aérosol (A) dans le volume d'accumulation (V) fixe étant, à la pression (p₂ = p_{A}) qui y est prédéfinie et qui est maintenue constante, fourni de telle sorte que l'évacuation de l'aérosol (A), commandée selon les besoins, à partir du volume d'accumulation (V), est compensée rapidement par une amenée dans le volume d'accumulation (V), commandée en fonction de l'évacuation intermittente, du premier constituant (F1) gazeux et du deuxième constituant (F2') liquide qui sont dans le rapport quantitatif (F1/F2') fixe prédéfini l'un par rapport à l'autre, et en ce que l'aérosol (A), pour l'évaporation du deuxième constituant (F2') liquide, ou bien le fluide (F) gazeux obtenu est ensuite chauffé indirectement sur la suite de son chemin d'écoulement, et le mélange gazeux (G) obtenu finalement, formé du premier constituant (F1) gazeux et du deuxième constituant (F2" ; H2O2") sous forme de vapeur, est conduit à sa destination finale.

20. Procédé selon la revendication 19,
**caractérisé en ce que**
l'aérosol (A) est chauffé au moyen de vapeur surchauffée (ST), en particulier de vapeur d'eau dans l'état de saturation.

21. Procédé selon la revendication 19 ou 20,
**caractérisé en ce que**
le chauffage s'effectue avec une température superficielle de 105 °C maximum.

22. Procédé selon une des revendications 19 à 21,
**caractérisé en ce que**
le premier constituant (F1) gazeux est préchauffé avant l'ajout du deuxième constituant (F2') pour mixage.

23. Procédé selon la revendication 22,
**caractérisé en ce que**
le préchauffage s'effectue à une température ϑ₁ = ϑ_{ΛR} (ϑ_{ΛS}) qui est supérieure à la température ultérieure ϑ₃ = ϑ_{G} du mélange gazeux (G), de préférence à ϑ₁ = 130 °C.

24. Procédé selon une des revendications 19 à 23,
**caractérisé en ce que**
la valeur effective du rapport quantitatif (F1/F2') distribuée sur le lieu d'utilisation du mélange gazeux (G) est déterminée par des techniques de mesure, et cette valeur de mesure est utilisée pour le préréglage des flux volumiques, conduits au volume d'accumulation (V), du premier constituant (F1) gazeux et du deuxième constituant (F2') liquide.

25. Procédé selon une des revendications 19 à 24,
**caractérisé en ce que,**
le flux volumique du premier constituant (F1) gazeux est déterminé pendant une première durée (Δt) prédéterminée de l'utilisation du mélange gazeux (G), et le flux volumique du deuxième constituant (F2') liquide est mesuré, **en ce que**, à partir de ces valeurs, la valeur effective du rapport quantitatif (F1/F2') est calculée, et **en ce qu'**un signal d'alarme et/ou d'arrêt est généré en cas d'écart au rapport quantitatif fixe prédéfini (valeur théorique) (F1/F2') égal à un intervalle de tolérance prédéfini.

26. Procédé selon une des revendications 19 à 25,
**caractérisé en ce que,**
pour une variation prédéfinie du niveau dans un réservoir de stockage (4) pour le deuxième constituant (F2') liquide, une deuxième durée (Δt_{F}) nécessaire pour cela est mesurée et cette dernière est utilisée pour le contrôle du flux volumique mesuré du deuxième constituant (F2') liquide et/ou du rapport quantitatif (F1/F2') (valeur théorique) fixe prédéfini.

27. Utilisation d'un dispositif selon une ou plusieurs des revendications 1 à 18 dans une machine d'emballage pour la stérilisation du moyen d'emballage ou de la zone d'emballage limitée par des parois de contenant.
